(19) Europäisches Patentamt
European Patent Office
Office européen des brevets

(11) Veröffentlichungsnummer: **0 222 322 B1**

# (12) EUROPÄISCHE PATENTSCHRIFT

(45) Veröffentlichungstag der Patentschrift: **06.05.92**

(51) Int. Cl.[5]: **C07D 501/46**, A61K 31/545

(21) Anmeldenummer: **86115367.4**

(22) Anmeldetag: **06.11.86**

Die Akte enthält technische Angaben, die nach dem Eingang der Anmeldung eingereicht wurden und die nicht in dieser Patentschrift enthalten sind.

## (54) Cephalosporinderivate und Verfahren zu ihrer Herstellung.

(30) Priorität: **11.11.85 DE 3539901**

(43) Veröffentlichungstag der Anmeldung:
**20.05.87 Patentblatt 87/21**

(45) Bekanntmachung des Hinweises auf die Patenterteilung:
**06.05.92 Patentblatt 92/19**

(84) Benannte Vertragsstaaten:
**AT BE CH DE FR GB IT LI LU NL SE**

(56) Entgegenhaltungen:
**EP-A- 0 098 609**
**DE-A- 3 137 854**

(73) Patentinhaber: **HOECHST AKTIENGESELLSCHAFT**
**Postfach 80 03 20**
**W-6230 Frankfurt am Main 80(DE)**

(72) Erfinder: **Lattrell, Rudolf, Dr.**
**Heuhohlweg 6h**
**W-6240 Königstein(Taunus)(DE)**
Erfinder: **Dürckheimer, Walter, Dr.**
**Im Lerchenfeld 45**
**W-6234 Hattersheim am Main(DE)**
Erfinder: **Kirrstetter, Reiner, Dr.**
**Altenhainer Strasse 8a**
**W-6233 Kelkheim (Taunus)(DE)**
Erfinder: **Seibert, Gerhard, Dr.**
**Gläserweg 21**
**W-6100 Darmstadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 222 322 B1

Rank Xerox (UK) Business Services
(3.08/2.19/2.0)

## Beschreibung

Die Erfindung betrifft neue Cephalosporinderivate, die in 3'-Stellung des Cephemrings durch einen über den Stickstoff gebundenen Imidazolring substituiert sind und die eine sehr gute antikmikrobielle Wirkung gegen gram-positive und gram-negative Bakterien besitzen und deshalb als Arzneimittel zur Behandlung von mikrobiellen Infektionen geeignet sind, sowie ein Verfahren zu ihrer Herstellung.

Gegenstand der Erfindung sind daher Cephalosporinderivate der allgemeinen Formel I

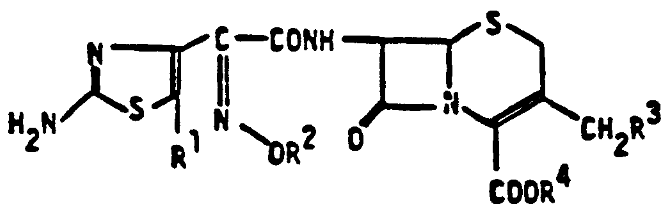

(I)

und deren physiologisch verträgliche Säureadditionssalze, worin

$R^1$ Wasserstoff oder Fluor, Chlor oder Brom

$R^2$ Wasserstoff,

$C_1$-$C_6$-Alkyl, das ein- oder mehrfach, gleich oder verschieden substituiert sein kann durch Fluor, Phenyl, 2- oder 3- oder 4-Tolyl, 2- oder 3- oder 4-Chlorphenyl, 1,3-Thiazol-4-yl, Imidazol-1-yl, $C_1$-$C_4$-Alkylthio, $C_1$-$C_4$-Alkoxy, Nitril oder Carbamoyl, worin die Aminogruppe ein- oder zweifach, gleich oder verschieden substituiert sein kann durch $C_1$-$C_6$-Alkyl, Hydroxy-($C_1$-$C_2$)-alkyl, Hydroxy oder Methoxy;

$C_2$-$C_6$-Alkenyl, das gegebenenfalls ein- oder mehrfach, gleich oder verschieden substituiert sein kann durch Chlor oder Brom,

$C_2$-$C_6$-Alkinyl, das gegebenenfalls substituiert sein kann durch Halogen,

$C_3$-$C_7$-Cycloalkyl, das gegebenenfalls substituiert sein kann durch Halogen,

$C_4$-$C_7$-Cycloalkenyl

$C_3$-$C_7$-Cycloalkylmethyl,

die Gruppen $-CH_2=CH-R^7$ oder

$$-(CH_2)_n-(\overset{R^5}{\underset{R^6}{C}})_m-R^7,$$

worin m und n jeweils für 0 oder I steht

und

$R^5$ und $R^6$ gleich oder verschieden sein können und Wasserstoff, Phenyl oder eine $C_1$-$C_{11}$-Alkylgruppe bedeuten, oder zusammen mit dem Kohlenstoff, an das sie gebunden sind, eine Methylen- oder eine $C_3$-$C_7$-Cycloalkylidengruppe bilden,

$R^7$ eine HOOC- oder $C_1$-$C_4$-AlkylOOC-Gruppe,

$R^3$ einen

Imidazol-1-yl-Rest- 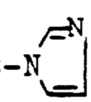 ,

der ein- oder mehrfach, gleich oder verschieden substituiert sein kann durch $C_1$-$C_6$-Alkyl, das ein- oder mehrfach substituiert sein kann durch Hydroxy, Acetoxy, Carbamoyloxy, Chlor, Carboxy, $C_1$-$C_6$-Alkyloxycarbonyl, Formyl, $C_1$-$C_6$-Alkylcarbonyl, Cyano, Carbamoyl, $C_1$-$C_6$-Alkyloxy und wobei 2 benachbarte Alkylgruppen auch zu einem gegebenenfalls durch $C_1$-$C_6$-Alkyl, Halogen, Hydroxy, Oxo, Carboxy, Carbamoyl substituierten Di- bis Decamethylen-Ring geschlossen sein können, in dem ein C-Atom durch ein Sauerstoff- oder Schwefelatom ersetzt sein kann und auch eine oder

zwei Doppelbindungen enthalten sein können,
durch $C_2$-$C_6$-Alkenyl, das durch Hydroxy substituiert sein kann,
durch $C_2$-$C_6$-Alkinyl
durch $C_3$-$C_6$-Cycloalkyl
durch $C_3$-$C_6$-Cycloalkylmethyl
durch Phenyl oder Benzyl, die durch $C_1$-$C_4$-Alkyl, Fluor, Chlor, Hydroxy, $C_1$-$C_4$-Alkyloxy substituiert sein können,
durch $C_1$-$C_6$-Alkoxy oder durch $C_1$-$C_6$-Alkylthio,
durch Fluor, Chlor, Brom, Jod, Trifluormethyl, Cyano, Hydroxy oder Mercapto,
durch Carboxy, $C_1$-$C_6$-Alkoxycarbonyl oder Carbamoyl, das am Stickstoff ein- oder zweimal substituiert sein kann durch $C_1$-$C_6$-Alkyl, Hydroxy oder Methoxy; durch Carbazoyl, das am Stickstoff ein- oder zweimal substituiert sein kann durch $C_1$-$C_4$-Alkyl,
durch Nitro, Amino oder Di-($C_1$-$C_2$)-alkylamino
durch Formyl, $C_1$-$C_4$-Alkylcarbonyl oder Benzoyl,

$R^4$ Wasserstoff, $C_1$-$C_6$-Alkyl, Benzhydryl, Allyl, Propargyl, Methoxymethyl, $C_1$-$C_6$-Alkanoyloxy-$C_1$-$C_6$-alkyl, $C_1$-$C_6$-Alkoxycarbonyloxy-$C_1$-$C_6$-alkyl, Phthalidyl oder 5-Methyl-1,3-dioxolen-2-on-4-yl-methyl bedeutet und in der die $R^2O$-Gruppe in syn-Position steht.

Als besonders bevorzugt kommen beispielsweise die folgenden Substituenten in Betracht:

$R^1$ : Wasserstoff, Fluor, Chlor, Brom

$R^2$ : ein $C_1$-$C_4$-Alkylrest, wie z.B. Methyl, Ethyl, Propyl, Isopropyl, Butyl, der ein- oder mehrfach, vorzugsweise 1- bis 4-fach, insbesondere 1- bis 2-fach substituiert sein kann, durch Fluor, wie insbesondere Monofluormethyl, Difluormethyl, Trifluormethyl, 1,1,2,2-Tetrafluoräthyl, 1,1,1-Trifluoräthyl, 1,1,2,2-Tetrafluorpropyl;

durch Phenyl, 2- oder 3- oder 4-Tolyl, 2- oder 3- oder 4-Chlorphenyl, substituiertes Alkyl, insbesondere Benzyl;

durch 1,3-Thiazol-4-yl substituiertes Alkyl, insbesondere 1,3-Thiazol-4-yl-methyl oder durch Imidazolyl substituiertes Alkyl, wie z.B. Imidazol-1-yl-äthyl,

durch $C_1$-$C_4$-Alkylthio substituiertes Alkyl, wie insbesondere Methylthiomethyl; durch $C_1$-$C_4$-Alkyloxy substituiertes Alkyl, wie insbesondere Methyloxymethyl, Äthyloxymethyl, Äthyloxyäthyl; durch Nitril oder Carbamoyl substituiertes Alkyl, wobei die Aminogruppe auch noch ein- oder zweimal substituiert sein kann, beispielsweise durch $C_1$-$C_4$-Alkyl, wie insbesondere N-Methyl- oder N-Äthyl- oder N-Propylcarbamoylmethyl, N,N-Dimethyl-carbamoylmethyl, N-Hydroxy- oder N-Methoxy-carbamoyl-methyl, N-Methyl-N-hydroxycarbamoyl-methyl oder N-Hydroxymethyl-carbamoyl-methyl, $C_2$-$C_6$-Alkenylrest, wie insbesondere 2-Propenyl, 2-Butenyl, der ein- oder mehrfach, vorzugsweise 1- bis 2-fach substituiert sein kann durch Chlor oder Brom, wie insbesondere 3-Chlor-2-propenyl, 2-Brom-2-propenyl
$C_2$-$C_6$-Alkinyl, wie insbesondere Propargyl,
$C_3$-$C_7$-Cycloalkyl, wie insbesondere Cyclopropyl, Cyclobutyl, Cyclopentyl oder Cyclohexyl,
$C_3$-$C_7$-Cycloalkylmethyl, wie insbesondere Cyclopropyl- und Cyclobutylmethyl,
$C_4$-$C_7$-Cycloalkenyl, wie insbesondere Cyclopenten-1-yl;
die Gruppe

$$-(CH_2)_n-\underset{R^6}{\overset{R^5}{(C)}}{}_m-R^7,$$

wobei $R^7$ die Gruppe COOH, bedeutet und $R^5$ und $R^6$ gleich oder verschieden sein können und Wasserstoff, Phenyl; $C_1$-$C_4$-Alkyl,
wie z.B. Methyl, Ethyl, Propyl, Isopropyl, Butyl, sec. Butyl, vorzugsweise Methyl, Ethyl, insbesondere Methyl, bedeuten können oder
wobei $R^5$ und $R^6$ zusammen mit dem Kohlenstoffatom, an das sie gebunden sind, eine Methylengruppe oder eine $C_3$-$C_7$-Cycloalkylidengruppe bilden können, wie z.B. Cyclopropyl, Cyclobutyl, Cyclopentyl, Cyclohexyl, Cycloheptyl, vorzugsweise Cyclopropyl, Cyclobutyl, Cyclopentyl oder Cyclohexyl
m = 0 oder 1
n = 0 oder 1, wobei die Summe von m und n 1 oder 2 darstellt.

Bevorzugt Beispiele für die Gruppe

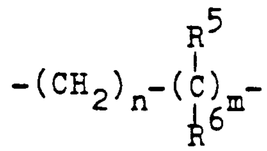

sind die folgenden:
Für den Fall, daß n = 0 und m = 1 ist:
$CH(CH_3)$, $C(CH_3)_2$, $CH(C_6H_5)$,

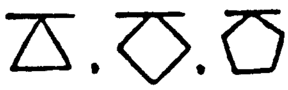

$CHC_2H_5$, $CH(CH_2)_9CH_3$;
für den Fall, daß m = 0 und n = 1 ist: $-CH_2-$ und falls n und m = 1 sind: $-CH_2-C(=CH_2)-$.

$R^3$: ein Imidazol-l-yl-Rest, der ein- oder mehrfach, vorzugsweise 1- bis 3-fach, insbesondere 1- bis 2-fach substituiert sein kann, beispielsweise durch gegebenenfalls 1- bis 2-fach, vorzugsweise gegebenenfalls 1-fach substituiertes $C_1$-$C_4$-Alkyl, wie insbesondere Methyl, Äthyl, Propyl, Isopropyl, n-Butyl, sec.-Butyl, ter.-Butyl,oder auch durch jeweils zwei Methyl- oder Äthylgruppen, durch drei Methylgruppen, oder auch durch Methyl in Kombination mit Äthyl, Propyl oder Isopropyl,
Hydroxy-$C_1$-$C_4$-alkyl, wie insbesondere Hydroxymethyl, Hydroxyäthyl, Hydroxypropyl, Hydroxyisopropyl, Hydroxybuthyl, Hydroxy-sec.-butyl oder Hydroxy-tert.-butyl, und wobei z.B. auch zwei Hydroxylgruppen am Alkylrest stehen können;
Acetoxy-$C_1$-$C_4$-alkyl, wie insbesondere Acetoxymethyl;
Carbamoyloxy-$C_1$-$C_4$-alkyl, wie insbesondere Carbamoyloxymethyl;
Chlor-$C_1$-$C_4$-alkyl, wie insbesondere Chlormethyl;
Carboxy-$C_1$-$C_4$-alkyl, wie insbesondere Carboxymethyl und Carboxyäthyl;
$C_1$-$C_4$-Alkoxycarbonyl-$C_1$-$C_4$-alkyl, wie insbesondere Methyloxycarbonylmethyl, Äthyloxycarbonylmethyl, Methyloxycarbonyläthyl;
Formyl-$C_1$-$C_4$-alkyl, wie insbesondere Formylmethyl;
$C_1$-$C_4$-Alkylcarbonyl-$C_1$-$C_4$-alkyl, wie insbesondere Methylcarbonylmethyl, Äthylcarbonylmethyl, Methylcarbonyläthyl und Äthylcarbonyläthyl;
Cyano-$C_1$-$C_3$-alkyl, wie insbesondere Cyanomethyl und Cyanoäthyl;
Carbamoyl-$C_1$-$C_4$-alkyl, wie insbesondere Carbamoylmethyl, Carbamoyläthyl;
$C_1$-$C_4$-Alkyloxy-$C_1$-$C_4$-alkyl, wie insbesondere Methyloxymethyl, Äthyloxymethyl, Propyloxymethyl, Isopropyloxymethyl, Methyloxyäthyl, Äthyloxyäthyl, Methyloxypropyl und Methyloxyisopropyl;
$C_3$-$C_4$-Alkenyl, wie insbesondere Allyl, 2-Methylallyl und Buten-3-yl, die auch noch durch Hydroxy substituiert sein können, wie insbesondere Hydroxyallyl und Hydroxybutenyl;
$C_3$-Alkinyl, wie insbesondere Propargyl,
$C_3$-$C_6$-Cycloalkyl und $C_3$-$C_6$-Cycloalkyl-methyl, wobei sich die Kohlenstoffzahl auf den Cycloalkylteil bezieht, wie insbesondere Cyclopropyl, Cyclobutyl, Cyclopentyl, Cyclohexyl, Cyclopropylmethyl und Cyclopentylmethyl, vorzugsweise Cyclopropyl und Cyclohexyl
Phenyl und Benzyl, die auch substituiert sein können, beispielsweise durch Chlor und Fluor, wie z.B. 4-Chlorbenzyl,4-Fluorphenyl;
$C_1$-$C_4$-Alkoxy, wie insbesondere Methoxy, Äthoxy, Propoxy, Isopropoxy, Butoxy, Isobutoxy und tert.-Butoxy, vorzugsweise Methoxy;
$C_1$-$C_4$-Alkylthio, wie insbesondere Methylthio, Äthylthio, Propylthio und Isopropylthio;
Fluor, Chlor, Brom, Jod, Trifluormethyl, Cyano, Hydroxy, Mercapto, Carboxy;
$C_1$-$C_4$-Alkoxycarbonyl, wie z.B. Methoxycarbonyl, Äthoxycarbonyl;
Carbamoyl, das am Stickstoffatom einmal oder zweimal substituiert sein kann durch $C_1$-$C_4$-Alkyl, wie insbesondere N-Methyl-, N-Äthyl-, N,N-Dimethylcarbamoyl, durch Hydroxy oder Methoxy, wie z.B. N-Hydroxy- oder N-Methoxycarbamoyl; Nitro; Amino; Dimethylamino;

Carbazoyl, das am Stickstoff ein- oder zweimal substituiert sein kann durch $C_1$-$C_4$-Alkyl, vorzugsweise Methyl, wie insbesondere $N^1$-Methyl-,$N^1$,$N^1$-Dimethylcarbazoyl;
Formyl; $C_1$-$C_4$-Alkylcarbonyl, insbesondere Acetyl und Propionyl; Benzoyl.

Stellt $R^3$ einen Imidazol-l-yl-Rest dar, der duch zwei benachbarte, zu einem Di- bis Decamethylen-Ring, vorzugsweise zu einem Tri- bis Pentamethylen-Ring, geschlossene Alkylgruppen substituiert ist, wobei in diesen ankondensierten Ringen ein C-Atom auch durch ein Sauerstoff- oder Schwefelatom ersetzt sein kann und auch ein oder zwei Doppelbindungen enthalten sein können, so kommen hierfür beispielsweise in Frage:

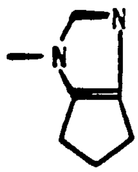

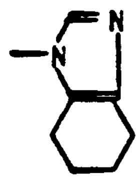

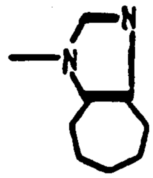

$R^4$ Wasserstoff, $C_1$-$C_4$-Alkyl, wie insbesondere Methyl, Äthyl, tert.-Butyl; Benzhydryl, Allyl, Propargyl, Methoxymethyl, $C_1$-$C_5$-Alkanoyloxy-$C_1$-$C_3$-alkyl, wie insbesondere Acetoxymethyl, Propionyloxymethyl, Isopropionyloxymethyl, n-Butyryloxymethyl, Isobutyryloxymethyl, Pivaloyloxymethyl, Isovaleryloxymethyl, 1-Acetoxyäthyl, 1-n-Propionyloxyäthyl, 1-Acetoxypropyl;
$C_1$-$C_5$-Alkoxycarbonyloxy-$C_1$-$C_3$-alkyl, wie insbesondere 1-Methoxycarbonyloxyäthyl, 1-Äthoxycarbonyloxyäthyl, 1-Isopropoxycarbonyloxyäthyl, Methoxycarbonyloxymethyl; Phthalidyl, 5-Methyl-1,3-dioxolen-2-on-4-yl-methyl.

Gegenstand der Erfindung ist weiterhin ein Verfahren zur Herstellung von Verbindungen der Formel I und ihrer physiologisch verträglichen Säureadditionssalze, das dadurch gekennzeichnet ist, daß man

a) eine Verbindung der allgemeinen Formel II

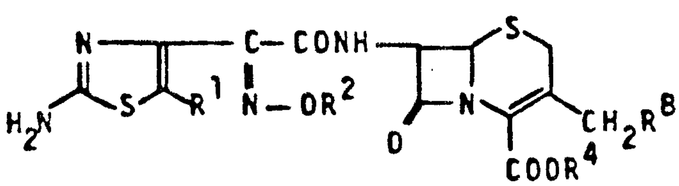

(II)

oder deren Salze, worin $R^1$, $R^2$ und $R^4$ die in Formel I genannte Bedeutung haben, die Aminogruppe auch geschützt sein kann, und $R^8$ eine durch Imidazol oder dasjenige Imidazolderivat, das den Resten $R^3$ der Formel I entspricht, austauschbare Gruppe bedeutet, mit Imidazol oder diesem Imidazolderivat umsetzt,

α) eine gegebenenfalls vorhandene Schutzgruppe abspaltet und

β) falls erforderlich, das erhaltene Produkt in ein physiologisch verträgliches Säureadditionssalz überführt,

oder

b) eine Verbindung der allgemeinen Formel III

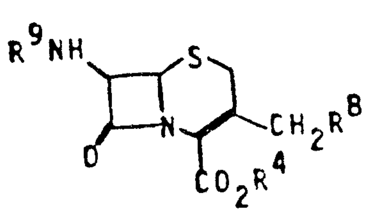

(III)

worin $R^4$ und $R^8$ die vorstehend für die Formel II genannte Bedeutung haben und $R^9$ für Wasserstoff oder eine Aminoschutzgruppe steht, mit Imidazol oder dem Imidazolderivat, das dem in Formel I

definierten Rest $R^3$ zugrunde liegt, umsetzt unter Bildung der Verbindung dei allgemeinen Formel IV,

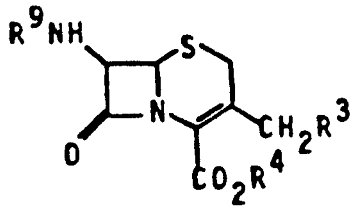

(IV)

in der $R^3$, $R^4$ und $R^9$ die oben genannte Bedeutung haben und

α) eine gegebenenfalls Vorhandene Aminoschutzgruppe abspaltet und

β) die Verbindung IV, worin $R^9$ Wasserstoff bedeutet, entweder als solche oder in Form eines reaktionsfähigen Derivates mit einer 2-syn-Oxyiminoessigsäure der allgemeinen Formel V,

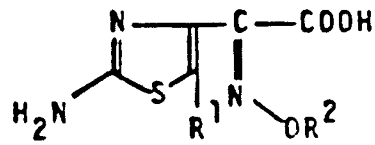

(V)

worin $R^1$ und $R^2$ die genannte Bedeutung besitzen und die Aminogruppe auch in geschützter Form vorliegen kann, oder mit einem an der Carboxylgruppe aktivierten Derivat dieser Verbindung umsetzt und

α) eine gegebenenfalls vorhande Schutzgruppe abspaltet,

β) falls erforderlich, das erhaltene Produkt der allgemeinen Formel I in ein physiologisch verträgliches Säureadditionssalz überführt und

γ) - falls Ester erhalten werden sollen - die nach den Verfahrensvarianten a) oder b) erhaltenen Verbindungen der Formel I, in der $R^4$ für Wasserstoff steht, in an sich bekannter Weise in die unter $R^4$ angegebenen Ester überführt.

Soll die Herstellung der Verbindung der allgemeinen Formel I nach der Verfahrensvariante a) durch nucleophilen Austausch von $R^8$ in den Verbindungen der allgemeinen Formel II durch Imidazol oder eines der angegebenen Imidazolderivate erfolgen, so kommen als Reste $R^8$ insbesondere in Betracht Acyloxyreste mit niederen aliphatischen Carbonsäuren, vorzugsweise mit 1 bis 4 C-Atomen, wie z.B.: Acetoxy oder Propionyloxy, insbesondere Acetoxy, die gegebenenfalls substituiert sein können, wie z.B.: Chloracetoxy oder Acetylacetoxy. Für $R^8$ kommen auch andere Gruppen in Betracht, wie beispielsweise Carbamoyloxy oder ein Halogenatom, wie z.B. Chlor, Brom oder Jod.

Die nucleophile Austauschreaktion an Verbindungen der allgemeinen Formel II, kann so erfolgen, daß die Reaktion in Gegenwart von Imidazol oder der den Resten $R^3$ entsprechenden Imidazolderivaten und von Tri-$C_1$-$C_4$-alkyljodsilanen, wie z.B. Trimethyl- oder Triäthyljodsilan, vorgenommen wird. Dabei kann so verfahren werden, daß die Verbindung II wobei $R^8$ für Acetoxy steht, zuerst mit Trimethyljodsilan nach den im folgenden genannten Reaktionsbedingungen zur Reaktion gebracht wird, die gebildete Verbindung II mit $R^8$ = J isoliert und anschließend mit Imidazol oder dem Imidazolderivat umgesetzt wird, oder die 3-$CH_2$J-Verbindung in situ durch Zugabe des Imidazols bzw. Imidazolderivates zur Reaktion gebracht wird. Statt Trimethyljodsilan kann beispielsweise auch eine Reaktionsmischung aus Jod und Hexamethyldisilan, die vorher bei Temperaturen zwischen etwa 60 und 120 °C in literaturbekannter Weise zur Reaktion gebracht wurden, wobei Trimethyljodsilan entsteht, verwendet werden. Statt Trimethyljodsilan kann mit denselben guten Ergebnis auch Triethyljodsilan, das in literaturbekannter Weise hergestellt wird, verwendet werden.

Die Reaktion wird ausgeführt bei Temperaturen zwischen etwa -5° und +100°C, vorzugsweise zwischen +10°C und +80°C. Geeignete inerte aprotische Lösungsmittel sind z.B. chlorierte Kohlenwasserstoff, wie Methylenchlorid, Chloroform, Dichlorethan, Trichlorethan, Tetrachlorkohlenstoff oder Niederalkylnitrile, wie Acetonitril oder Propionitril oder Frigene; insbesondere ist Methylenchlorid ein hervorragendes Lösungsmittel.

Das dem Rest $R^3$ entsprechende Imidazol bzw. Imidazolderivat wird in mindestens stöchiometrischer Menge bis zu einem zwanzigfachen Überschuß zugegeben, vorzugsweise wird mit solchen Mengen gearbeitet, daß die freiwerdende Jodwasserstoffmenge gebunden wird und noch mindestens 1 Mol, vorzugsweise 1,5 - 5 Mol Imidazols bzw. Imidazolderivates für die Substitution zur Verfügung stehen.

Da neben der auszutauschenden Gruppe $R^8$ in der Ausgangsverbindung II auch andere funktionelle Gruppen, wie z.B. die Aminogruppe oder die Carboxylgruppe, mit Trimethyljodsilan reagieren, wird letzteres in mindestens doppeltem bis zu fünfzehnfachen, vorzugsweise in drei- bis zehnfachem Überschuß zugegeben.

Derartige funktionelle Gruppen können auch durch Zugabe eines Silylierungsmittels wie z.B. Bistrimethylsilylacetamid, N-Methyl-N-trimethylsilyltrifluoracetamid, Bistrimethylsilyltrifluoracetamid, Trimethylchlorsilan, Hexamethyldisilazan, Bistrimethylsilylharnstoff, vorsilyliert werden, entweder ohne oder in Gegenwart einer Base, vorzugsweise des gewünschten, der Gruppe $R^3$ zugrundeliegenden Imidazols bzw. Imidazolderivates in den vorstehend beschriebenen Mengen. Anschließend wird Trimethyljodsilan in mindestens stöchiometrischer Menge oder auch im Überschuß, vorzugsweise in einem doppelten bis zu einem zehnfachen Überschuß zugegeben.

Liegt die Aminogruppe in den Formeln II und V in geschützter Form vor, so eignen sich als Aminoschutzgruppen z.B. gegebenenfalls substituiertes Alkyl, wie beispielsweise tert.-Butyl, tert.-Amyl; Benzyl, p-Methoxybenzyl, Trityl, Benzhydryl, vorzugsweise Trityl; Trialkylsilyl, wie beispielsweise Trimethylsilyl; gegebenenfalls substituiertes aliphatisches Acyl, wie z.B. Formyl, Chloracetyl, Bromacetyl, Trichloracetyl und Trifluoracetyl, vorzugsweise Formyl; oder gegebenenfalls substituiertes Alkoxycarbonyl, wie beispielsweise Trichloräthoxycarbonyl, Benzyloxycarbonyl oder tert.-Butyloxycarbonyl, vorzugsweise tert.-Butyloxycarbonyl und Benzyloxycarbonyl; oder Dimethylaminomethylen.

Die Schutzgruppe kann nach der Austauschreaktion in an sich bekannter Weise abgespalten werden, z.B. die Tritylgruppe mittels einer Carbonsäure, wie z.B. Essigsäure, Trifluoressigsäure, Ameisensäure, oder Benzyloxycarbonylgruppe hydrogenolytisch.

Die Reaktionsprodukte der Formel I können beispielsweise nach Zugabe von Wasser oder wäßrigen Mineralsäuren, z.B. verdünnter HCl, HBr, HJ oder $H_2SO_4$, aus der wäßrigen Phase in üblicher Weise, z.B. durch Gefriertrocknen der Wasserphase, Chromatographie oder Fällung durch Zugabe von organischen Lösungsmitteln, isoliert werden. Vorzugsweise werden die Reaktionsprodukte durch Ausfällen aus der Reaktionslösung in Form eines schwerlöslichen Salzes, beispielweise eines Hydrojodidsalzes, isoliert.

Für den Fall, daß $R^8$ für eine Carbamoyloxygruppe steht, wird die Austauschreaktion analog durchgeführt. Steht $R^8$ für Brom, so erfolgt der Austausch in literaturbekannter Weise.

Nach der Verfahrensvariante b) werden die Verbindungen der allgemeinen Formel I durch Acylierung von Verbindungen der allgemeinen Formel IV oder deren Additionssalze, beispielsweise mit Chlorwasserstoffsäure, Bromwasserstoffsäure, Salpetersäure, Schwefelsäure, Phosphorsäure oder einer organischen Säure, wie z.B. Methansulfonsäure oder Toluolsulfonsäure, mit Carbonsäuren der allgemeinen Formel V oder mit einem reaktionsfähigen Derivat einer solchen Säure erhalten.

Es ist dabei nicht erforderlich, die Verbindungen der allgemeinen Formel IV zu isolieren. Die Reaktion kann auch so durchgeführt werden, daß man Verbindungen der allgemeinen Formel III, beispielsweise 7-Aminocephalosporansäure oder 3-Jodmethyl-7-amino-ceph-3-em-4-carbonsäure oder deren reaktionsfähige Derivate in einem geeigneten Lösungsmittel mit dem dem Rest $R^3$ in der allgemeinen Formel IV entsprechenden Imidazol bzw. Imidazolderivat umsetzt und die entstandene Verbindung der allgemeinen Formel IV in situ zu den Verbindungen der allgemeinen Formel I acyliert. Erfindungsgemäß werden bei dieser Reaktion Ausgangsverbindungen der allgemeinen Formel III, in denen $R^8$ für Jod steht, eingesetzt. Geeignete Lösungsmittel sind chlorierte Kohlenwasserstoffe, wie z.B.: Methylen-chlorid und Chloroform; Ether, wie z.B.: Diethylether, Tetrahydrofuran, Dioxan; Acetonitril und Amide, wie vorzugsweise Dimethylformamid und Dimethylacetamid. Es kann sich auch als vorteilhaft erweisen, Gemische der genannten Lösungsmittel zu verwenden.

Liegt in den Verbindungen der allgemeinen Formel III ein reaktionsfähiges Derivat vor, so kommen insbesondere Silylderivate in Betracht, die bei der Umsetzung von Verbindungen der allgemeinen Formel III mit Silylverbindungen, wie z.B. Trimethylchlorsilan, Bis-(trimethylsilyl)-trifluoracetamid usw. gebildet werden. Die dem Rest $R^3$ entsprechende Base wird in mindestens stöchiometrischer Menge, bis zu einem zehnfachen Überschuß, vorzugsweise 1,5 bis 5 Äquivalente verwendet. Die Reaktion wird bei Temperaturen zwischen etwa -50 und +100 °C, vorzugsweise zwischen +20 und +50 °C ausgeführt.

Die Verbindungen der allgemeinen Formel IV können auch aus Verbindungen der allgemeinen Formel III, wobei $R^8$ für Acetoxy steht, in analoger Weise wie vorstehend für die Verbindungen der allgemeinen Formel II beschrieben, dargestellt werden.

Werden die Carbonsäuren der allgemeinen Formel V sowie ihre an der Aminogruppe geschützten Derivate selbst als Acylierungsmittel eingesetzt, so wird zweckmäßig in Gegenwart eines Kondensationsmittels, beispielsweise eines Carbodiimids, wie beispielsweise N,N'-Dicyclohexylcarbodiimid gearbeitet.

Die Aktivierung von Carbonsäuren der allgemeinen Formel V kann in besonders günstiger Weise durch Behandlung mit bestimmten Carbonsäureamiden und beispielsweise Phosgen, Phosphorpentachlorid, Tosylchlorid, Thionylchlorid oder Oxalylchlorid erfolgen, wie sie z.B. in der deutschen Patentschrift 28 04 040 beschrieben wird.

Als aktivierte Derivate der Carbonsäuren der allgemeinen Formel V eignen sich insbesondere auch Halogenide, vorzugsweise Chloride, die in an sich bekannter Weise durch Behandlung mit Halogenierungsmittel, wie z.B. Phosphorpentachlorid, Phosgen oder Thionylchlorid unter für die Cephalosporinchemie literaturbekannten, schonenden Reaktionsbedingungen erhalten werden.

Als aktivierte Derivate der Carbonsäuren der allgemeinen Formel V eignen sich ferner die Anhydride und gemischten Anhydride, Azide, aktivierten Ester und Thioester. Als gemischte Anhydride sind besonders geeignet solche mit niederen Alkansäuren, wie z.B. Essigsäuren und besonders bevorzugt solche mit substituierten Essigsäuren, wie z.B. Trichloressigsäure, Pivalinsäure und Cyanessigsäure. Besonders geeignet sind aber auch die gemischten Anhydride mit Kohlensäurehalbestern, die man beispielsweise durch Umsetzung der Carbonsäuren der Formel V, in denen die Aminogruppe geschützt ist, mit Chlorameisensäurebenzylester, -p-nitrobenzylester, -iso-butylester, -ethylester oder allylester gewinnt.

Als aktivierte Ester eignen sich vorzugsweise diejenigen mit p-Nitrophenol, 2,4-Dinitrophenol, Methylcyanhydrin, N-Hydroxysuccinimid und N-Hydroxyphthalimid, insbesondere diejenigen mit 1-Hydroxybenzotriazol und 6-Chlor-1-hydroxybenzotriazol. Besonders bevorzugte Thioester sind beispielsweise diejenigen mit 2-Mercaptobenzothiazol und 2-Mercaptopyridin. Die aktivierten Derivate können als isolierte Substanzen aber auch in situ umgesetzt werden.

Im allgemeinen erfolgt die Umsetzung der Cephemderivate der allgemeinen Formel IV mit einer Carbonsäure der allgemeinen Formel V oder einem aktivierten Derivat derselben in Gegenwart eines inerten Lösungsmittels. Insbesondere eignen sich chlorierte Kohlenwasserstoffe, wie vorzugsweise Methylenchlorid und Chloroform; Äther, wie z.B. Diäthyläther, vorzugsweise Tetrahydrofuran und Dioxan; Ketone, wie vorzugsweise Aceton und Butanon; Amide, wie vorzugsweise Dimethylformamid und Dimethylacetamid, oder Pyridin. Es kann sich auch als vorteilhaft erweisen, Gemische der genannten Lösungsmittel zu verwenden. Dies ist oftmals dann der Fall, wenn die Cephemverbindung der allgemeinen Formel IV mit einem in situ erzeugten aktivierten Derivat einer Carbonsäure der Formel V umgesetzt wird.

Die Umsetzung von Cephemverbindungen der Formel IV mit Carbonsäuren der Formel V bzw. deren aktivierten Derivaten kann in einem Temperaturbereich von etwa -80 bis etwa +80 ° C, vorzugsweise zwischen etwa -20 ° C und Raumtemperatur erfolgen.

Die Reaktionsdauer hängt von den Reaktanten, der Temperatur und dem Lösungsmittel bzw. dem Lösungsmittelgemisch ab und liegt normalerweise zwischen 1/4 und etwa 72 Stunden.

Die Reaktion mit Säurehalogeniden kann gegebenenfalls in Gegenwart eines säurebindenen Mittels zur Bindung des freigesetzten Halogenwasserstoffs durchgeführt werden. Als solche eignen sich insbesondere tertiäre Amine, wie z.B. Triäthylamin oder Dimethylanilin, anorganische Basen, wie z.B. Kaliumcarbonat oder Natriumcarbonat, Alkylenoxide, wie z.B. Propylenoxid. Auch die Anwesenheit eines Katalysators, wie z.B. von Dimethylaminopyridin, kann gegebenenfalls von Vorteil sein. Liegt in den Verbindungen der allgemeinen Formel IV die Aminogruppe in Form eines reaktionsfähigen Derivats vor, so kann es sich um ein solches handeln, wie aus der Literatur für Amidierungen bekannt ist. So kommen beispielsweise Silylderivate in Betracht, die bei der Umsetzung von Verbindungen der allgemeinen Formel IV mit einer Silylverbindung gebildet werden, wie z.B. Trimethylchlorsilan oder Bis-(trimethylsilyl)-acetamid. Wird die Umsetzung mit einer solchen, an der Aminogruppe aktivierten Verbindung durchgeführt, so ist es zweckmäßig, die Reaktion in einem inerten Lösungsmittel, wie z.B. Methylenchlorid, Tetrahydrofuran oder Dimethylformamid durchzuführen.

Sollen Verbindungen der allgemeinen Formel I erhalten werden in der $R^4$ für $C_1$-$C_6$-Alkanoyloxy-$C_1$-$C_6$-alkyl, $C_1$-$C_6$-Alkoxycarbonyloxy-$C_1$-$C_6$-alkyl, Phthalidyl oder 5-Methyl-1,3-dioxolen-2-on-4-ylmethyl steht, so setzt man in an sich bekannter Weise die Verbindungen der allgemeinen Formel I, in denen $R^4$ = Wasserstoff bedeutet, mit einer Verbindung der allgemeinen Formel VI

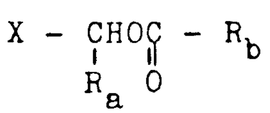

(VI)

worin $R_a$ = Wasserstoff oder eine $C_1$-$C_5$-Alkylgruppe, $R_b$ = eine $C_1$-$C_6$-Alkyl oder eine $C_1$-$C_6$-Alkyloxygruppe bedeutet, oder VII oder VIII

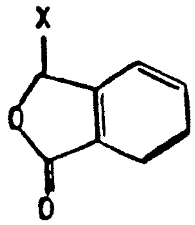 (VII) 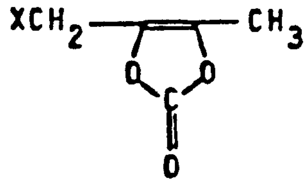 (VIII)

worin - wie auch in der Formel VI - X für Halogen, vorzugsweise Chlor, Brom oder Jod steht, um. Es werden Verfahren angewendet, wie sie für Veresterungsreaktionen bekannt sind. Steht $R^4$ für $C_1$-$C_6$-Alkyl, Benzhydryl, Allyl oder Propargyl, so werden die für diese Gruppen literaturbekannten Veresterungsmethoden angewandt. Schutzgruppen können in bekannter Weise wie oben beschrieben, entfernt werden.

Als physiologisch verträgliche Säureadditionssalze der Verbindungen der allgemeinen Formel I seien beispielsweise erwähnt solche mit Chlorwasserstoffsäure, Bromwasserstoffsäure, Salpetersäure, Phosphorsäure, Schwefelsäure oder organische Säuren, wie z.B. Methansulfonsäure, p-Toluolsulfonsäure oder Maleinsäure.

Die erfindungsgemäß eingesetzten Imidazole und anderen Ausgangsverbindungen sind literaturbekannt oder können nach literaturbekannten Verfahren erhalten werden.

Die erfindungsgemäß erhaltenen Verbindungen der allgemeinen Formel I und ihre physiologisch verträglichen Säureadditionssalze zeigen bemerkenswert gute antibakterielle Wirksamkeit sowohl gegen grampositive als auch gramnegative bakterielle Keime.

Auch gegen penicillinase- und cephalosporinase-bildende Bakterien sind die Verbindungen der Formel I unerwartet gut wirksam. Da sie zudem günstige toxikologische und pharmakologische Eigenschaften zeigen, stellen sie wertvolle Chemotherapeutika dar.

Die Erfindung betrifft somit auch Arznei-präparate zur Behandlung von mikrobiellen Infektionen, die durch einen Gehalt an einer oder mehreren der erfindungsgemäßen Verbindungen charkaterisiert sind.

Die erfindungsgemäßen Produkte können auch in Kombinationen mit anderen Wirkstoffen, beispielsweise aus der Reihe der Penicilline, Cephalosporine oder Aminoglykoside zur Anwendung kommen.

Die Verbindungen der allgemeinen Formel I und ihre physiologischen verträglichen Säureadditionssalze können oral, intramuskulär oder intravenös verabfolgt werden.

Arzneipräparate, die eine oder mehrere Verbindungen der allgemeinen Formel I als Wirkstoff enthalten, können hergestellt werden, indem man die Verbindungen der Formellmit mehreren pharmakologisch verträglichen Trägerstoffen oder Verdünnungsmittel, wie z.B. Füllstoffen, Emulgatoren, Gleitstoffen, Geschmackskorrigentien, Farbstoffen oder Puffersubstanzen vermischt und in eine geeignete galenische Zubereitungsform bringt, wie beispielsweise Tabletten, Dragees, Kapseln oder eine zur parenteralen Applikation geeignete Suspension oder Lösung.

Als Träger- oder Verdünnungsmittel seien beispielsweise erwähnt Traganth, Milchzucker, Talkum, Agar-Agar, Polyglykole, Äthanol und Wasser. Puffersubstanzen sind beispielsweise organische Verbindungen, wie z.B. N,N'-Dibenzyläthylendiamin, Diäthanolamin, Äthylendiamin, N-Methylglucamin, N-Benzylphenäthylamin, Diäthylamin, Tris(hydroxymethyl)aminomethan, oder anorganische Verbindungen wie z.B. Phosphatpuffer, Natriumbicarbonat, Natriumcarbonat. Für die parenterale Applikation kommen vorzugsweise Suspensionen oder Lösungen in Wasser mit oder ohne Puffersubstanzen in Betracht. Es ist auch möglich, die Wirkstoffe als solche ohne Träger- oder Verdünnungsmittel in geeigneter Form, beispielsweise in Kapseln, zu applizieren.

Geeignete Dosen der Verbindungen der allgemeine Formel I oder ihrer physiologisch verträglichen Säureadditionssalze liegen bei etwa 0,4 bis 20 g/Tag, vorzugsweise bei 0,5 bis 4 g/Tag, für einen Erwachsenen von etwa 60 kg Körpergewicht.

Es können Einzel- oder im allgemeinen Mehrfachdosen verabreicht werden, wobei die Einzeldosis den Wirkstoff in einer Menge von etwa 50 bis 1000 mg, vorzugsweise von etwa 100 bis 500 mg, enthalten kann.

Die folgenden Ausführungsbeispiele für erfindungsgemäß herstellbare syn-Verbindungen dienen zur weiteren Erläuterung der Erfindung, schränken sie jedoch nicht darauf ein.

Beispiel 1:

7- [ 2-(2-Aminothiazol-4-yl)-2-syn-methoxyimino-acetamido] -3- [ (2-methylimidazol-1-yl)methyl ]-ceph-3-em-4-carbonsäure

Verfahren a)

11,8 g (126 mmol) 7- [ 2-(2-Aminothiazol-4-yl)-2-syn-methoxyimino-acetamido ]-cephalosporansäure werden in 50 ml Chloroform suspendiert, mit 16,25 ml (88 mmol) N-Methyl-N-trimethylsilyltrifluoracetamid (MSTFA) versetzt und 1 Stunde bei Raumtemperatur gerührt. Es wird auf 18°C gekühlt, 10 ml (79 mmol) Jodtrimethylsilan zugegeben und weitere 20 Minuten bei Raumtemperatur (23°C) gerührt. Es wird im Vakuum eingeengt, der ölige Rückstand in 50 ml Acetonitril gelöst und 2,1 ml Tetrahydrofuran zugegeben. Diese Lösung wird auf einmal zu einem Gemisch aus 2,63 g (32 mmol ) 2-Methylimidazol und 11 ml (59 mmol) MSTFA in 26 ml Acetonitril gegeben und die dunkelgefärbte Lösung 1,5 Stunden bei Raumtemperatur belassen. Unter Eiskühlung und Umschütteln werden sodann 3,1 ml Wasser zugegeben. Der gebildete Niederschlag wird nach 10 Minuten abgesaugt, zweimal mit Acetonitril, sodann mit Äthanol und Äther gewaschen und getrocknet. Ausbeute an Hydrojodidsalze der Titelverbindung : 13,8 g (88 % d. Th). Das rohe Hydrojodid wird in wässriger Natriumbicarbonatlösung gelöst und über Kieselgel (5 x 55 cm-Säule) mit Aceton:Wasser (5:1) chromatographiert. Nach Gefriertrocknung der Fraktionen 8-14 (800 ml) erhält man 1,1 g (9 %) Δ 2-Verbindung, die Fraktionen 15-24 (1,2 l) ergeben 7,5 g (60 %) der Titelbindung als gelblich gefärbten amorphen Feststoff.

$^1$H-NMR(DMSO-$d_6$) δ = 2,33 (s, 3H, IM-$CH_3$); 3,02 und 3,26 (AB,J = 18Hz, 2H, $SCH_2$); 3,82 (s, 3H, $OCH_3$); 4,90 (AB, 2H, $CH_2N$); 5,02 (d, J = 5Hz, 6-H); 5,60 (dd, J = 5 und 9Hz, 7-H); 6,70 (s, 1H, Thiazol-H); 6,78 (d, J = 2Hz, 1 Imidazol-H); 7,20 (bs, 3H, $NH_2$ und 1 Imidazol-H); 9,52 ppm (d, J = 9Hz, 1H, CONH).

Verfahren b, Variante α

3,39 g (10 mmol) 7-Amino-3-jodmethyl-ceph-3-em-4-carbonsäure werden in 80 ml Tetrahydrofuran suspendiert und nach Zugabe von 5 ml (20 mmol) Bis(trimethylsilyl)acetamid (BSA) 1,5 Stunden bei Raumtemperatur gerührt. Unter Kühlen werden sodann 1,23 g (15 mmol) 2-Methylimidazol zugegeben und 4.5 Stunden bei Raumtemperatur (23°C) gerührt. Unter kühlen werden sodann 1 ml Wasser zugegeben, der gebildete Niederschlag abgesaugt und mit Äthanol, Aceton und Äther gewaschen. Man erhält 4,5 g Monohydrojodidsalz der 7-Amino-3[ (2-methylimidazol-1-yl)methyl ]-ceph-3-em-4-carbonsäure. Das rohe Salz wird in eine Lösung von 2,86 g (9 mmol) 2-(2-Aminothiazol-4-yl)-2-syn-methoxyiminoessigsäure-Hydroxybenztriazol-Aktivester in 60 ml N,N-Dimethylformamid und 2 ml Wasser eingetragen. Es wird 8 Stunden bei 5°C belassen. Die DMF-Lösung wird in 1 l Äther eingegossen, der ausgefallene Niederschlag in wässriger Natriumbicarbonatlösung gelöst und die dunkel gefärbte Lösung über eine "Lobar-C"-Kieselgelsäule (Fa. Merck-Darmstadt) mit Aceton : Wasser (5:1) chromatographiert. Nach Gefriertrocknen der Produktfraktionen erhält man 990 mg (23 % d. Th) eines amorphen Feststoffs. Er ist in allen Eigenschaften mit dem nach Verfahren a) erhaltenen Produkt identisch.

Verfahren b, Variante β

7-Amino-3-[ (2-methylimidazol-1-yl)methyl ]-ceph-3-em-4-carbonsäure-Monohydrojodid

2,96 g (10,8 mmol) 7-Aminocephalosporansäure und 10 ml (9,7 g, 37,8 mmol) N,O-Bis(trimethylsilyl)-trifluoracetamid (BSTFA) in 25 ml Methylendichlorid werden 1 Stunde unter Rückfluß gehalten. Die Lösung wird auf 20°C gekühlt und 4ml (28 mmol) Jodtrimethylsilan zugegeben. Es wird 20 Minuten bei Raumtemperatur gehalten, im Vakuum eingeengt und der ölige Rückstand in einem Gemisch aus 20 ml Acetonitril und 0,8 ml Tetrahydrofuran gelöst. Es wird auf 15°C gekühlt und eine Lösung von 1,07 g (13 mmol) 2-Methylimidazol und 5,0 ml BSTFA in 10 ml Acetonitril zugegeben. Die dunkelgefärbte Mischung wird 1,5 Stunden bei Raumtemperatur belassen und sodann unter Kühlung durch Zugabe von 1 ml Wasser hydrolysiert. Der gebildete Niederschlag wird nach 1 Stunde abgesaugt, zweimal mit Acetonitril, sodann mit Äthanol und Äther gewaschen und getrocknet, Man erhält 4,2 g (10 mmol, 92 % d. Th) der Titelverbindung.

NMR ($CF_3CO_2D$) : δ = 2,81 (s, 3H, $CH_3$); 3,60 (AB, 2H, $SCH_2$); 5,1 - 5,8 (m, 4H, 2Laktam-H und $CH_2N$); 7.2 - 7,55 ppm (m, 2 Imidazol-H)

Durch Acylierung mit 2-(2-Aminothiazol-4-yl)-2-synmethxyiminoessigsäure-Hydroxybenztriazol-Aktivester in

= DMF/$H_2O$ wird wie vorstehend in Variante α beschrieben 7- [ 2-(2-Aminothiazol-4-yl ) -2-syn-methoxyimino-acetamido] - 3- [ (2-methylimidazol-1-yl)methyl ] -ceph-3-em-4-carbonsäure nach Chromatographie als amorpher Feststoff erhalten. Die Verbindung ist in allen Eigenschaften mit der nach Verfahren a) erhaltenen indentisch.

Beispiel 2:

7- [ 2-(2-Aminothiazol-4-yl)-2-syn-(2-carboxyprop-2-yl-oxyimino)acetamido] -3- [ (2-methyl-imidazol-1-yl)-methyl ]-ceph-3-em-4-carbonsäure

1,1 g (2 mmol) 2-syn-(2-tert. Butyloxycarbonyl-prop-2-yl-oximino)-2-(2-tritylaminothiazol-4-yl)-essigsäure werden zusammen mit 0,32 g 1-Hydroxy-1H-benzotriazol-Hydrat und 0,5 g N,N'-Dicyclohexylcarbodiimid in 9 ml Dimethylformamid 3 Stunden bei Raumtemperatur gerührt. Vom ausgefallenen Dicyclohexylharnstoff wird filtriert und zur Lösung des Aktivesters 760 mg (1,8 mmol) 7-Amino-3-[ (2-methylimidazol-1-yl)methyl ] -ceph-3-em-4-carbonsäure-Monohydrojodid (Beispiel 1, Verfahren b, Variante β) gegeben. Nach 17 Stunden bei Raumtemperatur wird in 800 ml Äther eingerührt, das ausgefallene Produkt abgesaugt und im Vakuum getrocknet. Das Rohprodukt wird in 20 ml 90proz. Trifluoressigsäure gelöst. Nach 20 Minuten wird i. Vak. eingeengt, der ölige Rückstand zweimal mit Toluol abgedampft. Der Rückstand wird in wässriger Natriumbicarbonatlösung gelöst und über eine "Lobar B"-Kieselgelsäule (Fa. Merck, Darmstadt) mit Essigester/Isopropanol/Wasser (20:15:10) chromatographiert. Nach Gefriertrocknung der Produktfraktionen erhält man 306 mg (28 % d. Th) der Titelverbindung als hellgelben amorphen Feststoff.

$^1$H-NMR (DMSO-$d_6$) : δ = 1,42 (s, 3H, $CH_3$); 1,47 (s, 3H, $CH_3$); 3.00 und 3,18 (AB, J = 18Hz, 2H, $SCH_2$); 4,75 und 4,95 (AB, 2H, $CH_2W$), 4,99 (d, J = 5Hz, 6-H); 5,58 (dd, J = 5 und 8Hz, 7-H), 6,70 (s, 1H, Thiazol-H); 6,82 (s, 1 Imidazol-H); 7,15 (bs, 3H, $NH_2$ und 1 Imidazol-H); 10,55 ppm (d, J = 8Hz, 1H, CONH).

Analog Beispiel 1, Verfahren a) werden die nachfolgend aufgeführten Beispiele als amorphe Feststoffe erhalten, die der allgemeinen Formel I mit $R^4$ = Wasserstoff entsprechen und worin $R^1$, $R^2$ und $R^3$ die in der Tabelle 1 angegebene Bedeutung haben.

## Tabelle 1

| Beispiel | $R^1$ | $R^2$ | $R^3$ | $^1H$-NMR (Lösungsmittel): δ(ppm)* |
|---|---|---|---|---|
| 3 | H | $CH_3$ | 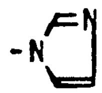 | ($CF_3COOD$) : 3,52 und3,80 (AB, J = 18 Hz, 2H, $SCH_2$); 4,24 (s, 3H, $OCH_3$); 5,20 und 5,70 (AB, J = 14Hz, $CH_2N$); 5,36 (d, J = 5Hz, 6-H) 6,11 (d, J = 5Hz, 7-H); 7,43 (s, 1H, Thiazol-H); 7,53; 7,66 und 8,92 (je ein bs, 3 Imidazol-H) |
| 4 | H | $CH_3$ | 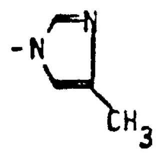 | ($CF_3COOH$) : 2,46 (s, 3H, $CH_3$); 3,50 und 3,75 (AB, J = 18Hz, 2H, $SCH_2$); 4,25 (s, 3H, $OCH_3$), 5,18 und 5,65 (AB, J = 14Hz, $CH_2N$); 5,35 (d, J = 5Hz, 6-H) 6,05 (d, J = 5Hz, 7-H); 7,35 (m, 2H, Thiazol- und Imidazol-H) 8,71 (bs,1 Imidazol-H) |
| 5 | H | $CH_3$ | 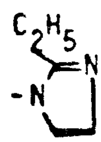 | ($CF_3COOD$) : 1,52 (t, 3H, $CH_2\underline{CH_3}$); 3,13 (q, 2H, $\underline{CH_2}CH_3$); 3,41 und 3,70 (AB, J = 18Hz, 2H, $SCH_2$); 4,24 (s, 3H, $OCH_3$); 5,20 und 5,58 (AB, J = 15Hz, $CH_2N$); 5,38 (d, J = 5Hz, 6-H); 6,08 (d, J = 5Hz, 7H); 7,2 - 7,55 (m, 3H, Thiazol-H und 2 Imidazol-H). |

**Tabelle 1**
**(Fortsetzung)**

| Beispiel | $R^1$ | $R^2$ | $R^3$ | $^1H$-NMR (Lösungsmittel): δ (ppm) = |
|---|---|---|---|---|
| 6 | H | $CH_3$ | 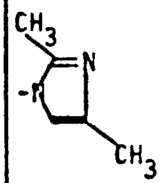 | ($CF_3CO_2D$): 2,70 (s, 3H, $CH_3$); 2,79 (s, 3H, $CH_3$); 3,42 und 3,70 (AB, J = 18Hz, 2H, $SCH_2$); 4,27 (s, 3H, $OCH_3$); 5,10 und 5,50 (AB, J = 15Hz, $CH_2N$); 5,37 (d, J = 5Hz, 6-H); 6,28 (d, J = 5Hz, 7-H); 7,15 (s, 1 Imidazol-H); 7,41 (s, 1 Thiazol-H). |
| 7 | Cl | $CH_3$ | 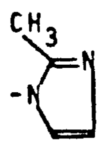 | (DMSO-$d_6$): 2,36 (s, 3H, $CH_3$); 3,10 und 3,30 (AB, J = 18Hz, 2H, $SCH_2$); 3,83 (s, 3H, $OCH_3$); 4,89 (AB, 2H, $CH_2N$); 5,05 (d, J = 5Hz; 6-H); 5,65 (dd, J = 5 und 8Hz, 7-H); 6,92 (bs, 1 Imidazol-H); 7,22 (bs, 1 Imidazol-H); 7,32 (bs, $NH_2$); 9,45 (d, J = 8Hz, CONH); |
| 8 | H | $CH_3$ | 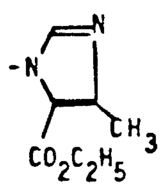 | (DMSO-$d_6$): 1,30 (t. 3H, $CH_2\underline{CH_3}$); 2,36 (s, 3H, $CH_3$); 3,20 (AB, 2H, $SCH_2$); 3,84 (s, 3H, $OCH_3$); 4,15 (q, 2H, $\underline{CH_2}CH_3$); 4,95 (AB, 2H, $CH_2N$); 5,05 (d, J = 5Hz, 6-H); 5,55 (dd, J = 5 und 8Hz, 7-H); 6,62 (s, 1 Thiazol-H); 7,96 (bs, 1-Imidazol-H) 9,43 (d, J = 8Hz, CONH). |
| 9 | H | $CH_3$ | 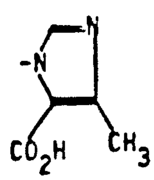 | (DMSO-$d_6$): 2,33 (s, 3H, $CH_3$); 3,35 (AB, 2H, $SCH_2$); 3,82 (s, 3H, $OCH_3$); 4,65 (AB, 2H, $CH_2N$); 4,92 (d, J = 5Hz, 6-H); 5,50 (dd, J = 5 und 8Hz, 7-H); 6,70 (s, 1 Thiazol-H); 7.05 - 7.35 (m, 3H, $NH_2$ und 1 Imidazol-H); 9,42 (d, J = 8Hz, CONH). |

Tabelle 1
(Fortsetzung)

| Beispiel | $R^1$ | $R^2$ | $R^3$ | $^1$H-NMR (Lösungsmittel):δ(ppm)= |
|---|---|---|---|---|
| 10 | H | $CH_2COOH$ | 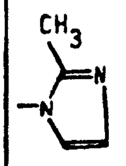 | ($DMSO-d_6$): 2,28 (s, 3H, $CH_3$); 3,05 (AB, 2H, $SCH_2$); 4,31 (bs, 2H, $OCH_2$); 4,80 (AB, 2H, $CH_2N$); 4,98 (d, J = 5Hz, 6-H); 5,56 (dd, J = 5 und 8Hz, 7-H); 6,70 (s, 1 Thiazol-H), 6,78 (d, 1 Imidazol-H); 7,0 - 7,3 (m, 1 Imidazol-H und $NH_2$); 11,10 (d, J = 8Hz, CONH); |
| 11 | H | $CH_3$ | 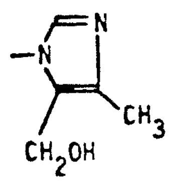 | ($CF_3CO_2D$): 2,47 (s, 3H, $CH_3$): 3,62 (AB, 2H, $SCH_2$); 4,22 (s, 3H, $OCH_3$): 5,00 (s, 2H, $CH_2OH$); 5,32 und 5,63 (AB, J = 14Hz, $CH_2N$); 5,33 (d, J = 5Hz, 6-H): 6.05 (d, J = 5Hz, 7-H): 7,40 (s, Thiazol-H); 8,72 (s, Imidazol-H); |
| 12 | H | $CH_3$ | 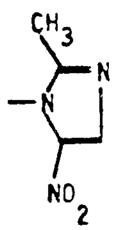 | ($CF_3CO_2D$): 2,42 (s, 3H, $CH_3$); 3,65 (AB, 2H, $SCH_2$); 4,25 (s, 3H, $OCH_3$); 5,22 (AB, 2H, $CH_2N$): 5,39 (d, J = 5Hz, 6-H); 6,20 (d, J = 5Hz, 7H): 7,43 (s, Thiazol-H); 7,50 (s, Imidazol-H); |

Beispiel 13:

7-[ 2-(2-Aminothiazol-4-yl)-2-syn-hydroxyimino-acetamido ] -3-[(2-methyl-imidazol-1-yl)methyl ]-ceph-3-em-4-carbonsäure

Aus 671 mg (1 mmol) 2-(2-Tritylaminothiazol-4-yl)-2-trityloxyiminoessigsäure, 160 mg 1-Hydroxy-1H-benzotriazolhydrat und 250 mg N,N'-Dicyclohexylcarbodiimid in 4,5 ml DMF wird analog Beispiel 2 eine Lösung des Aktivesters bereitet. Nach Zugabe von 380 mg (0,9 mmol) 7-Amino-3- [(2-methylimidazol-1-yl)-methyl ] -ceph-3-em-4-carbonsäure-Monohydrojodid wird 4 Stunden bei Raumtemperatur und 17 Stunden bei 5 ° C belassen. Vom ausgefallenen Dicyclohexylharnstoff wird filtriert, die Lösung i. Vak. eingeengt. Der ölige Rückstand wird in 20 ml 90proz. Trifluoressigsäure gelöst. Es wird 30 Minuten bei Raumtemperatur belassen, i. Vak. eingeengt und der Rückstand zweimal mit Toluol abgedampft. Der Rückstand wird sodann in Äther suspendiert, abgesaugt und mit Äther gewaschen. Ausbeute: 0,35 g. Dieses Rohprodukt wird in wässriger Natriumbicarbonatlösung gelöst und über eine "Lobar B"-Kieselgelsäule mit Essigester/Isopropanol/Wasser (20:15:10) chromatographiert. Nach Gefriertrocknung der Produktfraktionen erhält man 80 mg der Titelverbindung als gelblich gefärbten amorphen Feststoff.

$^1$H-NMR ($CF_3CO_2D$) : 2,80 (s, 3H, $CH_3$); 3,40 und 3,65 (AB, J = 18Hz, 2H, $SCH_2$); 5,05 - 5,72 (m, 3H, AB von $CH_2N$ und 6-H); 6,29 (d, J = 5Hz, 7-H); 7,3 - 7,6 (m, 3H, 2 Imidazol-H, 1 Thiazol-H);

Analog Beispiel 1, Verfahren b), Variante $\beta$) werden die nachfolgend aufgeführten Beispiele aus 7-Amino-3-[ (2-methylimidazol-1-yl)methyl ] -ceph-3-em-4-carbonsäure-Monohydrojodid und den entsprechenden HOBT-Aktivestern als amorphe Feststoffe erhalten, die der allgemeinen Formel I mit $R^1$ und $R^4$ = Wasserstoff und $R^3$ =

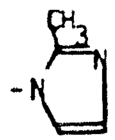

entsprechen und worin

$R^2$ die in der Tabelle 2 angegebene Bedeutung hat.

Tabelle 2

| Beispiel | $R^2$ | $^1H$-NMR (Lösungsmittel) : δ (ppm)= |
|---|---|---|
| 14 | $-C_2H_5$ | ($CF_3CO_2D$) : 1,45 (t, 3H, $CH_2CH_3$); 2,83 (s, 3H, $CH_3$); 3,43 und 3,73 (AB, J = 18Hz, 2H, $SCH_2$); 4,55 (q, 2H, $CH_2CH_3$); 5,22 und 5,55 (AB, J = 15Hz, $CH_2N$); 5,38 (d, 7 =5Hz, 6-H); 6,10 (d, J = 5Hz, 7-H); 7,2 - 7,6 (m, 3H, Thiazol-H und 2 Imidazol-H). |
| 15 | $-CH_2CONH_2$ | ($CF_3CO_2D$) : 2,83 (s, 3H, $CH_3$): 3,42 und 3,69 (AB, J = 18Hz, 2H, $SCH_2$): 5,02 (s, 2H, $CH_2CONH_2$); 5,1 - 5,7 (m, 3H, AB von $CH_2N$ und 6-H); 6,23 (d, J = 5Hz, 7-H): 7.2 - 7,65 (m, 3H, 2 Imidazol-H und 1 Thiazol-H): |
| 16 | $-CH_2CONHCH_3$ | (DMSOd$_6$): 2,31 (s,3H,$CH_3$); 2.63 (d, J=5Hz, 3H, $NHCH_3$); 3.05 und 3,29 (AB, J=17Hz, 2 H, $SCH_2$); 4.45 (s, 2H, $CH_2CONHCH_3$); 4.85 und 4.95 (AB, J=15Hz, $CH_2N$); 5.07 (d, J=5Hz, 6-H); 5,71 (dd, J=5 und 8Hz, 7-H); 6.82 (s, 1Thiazol-H); 6.9? (s, 1Imidazol-H); 7.22 (bs, 1Imidazol-H) 7.28 (bs, 2H, $NH_2$); 7.43 (d. J=5Hz. $NHCH_3$); 9.70 (d, J=8Hz, CONH). |

Tabelle 2
(Fortsetzung)

| Beispiel | $R^2$ | $^1$H-NMR (Lösungsmittel) : δ (ppm)= |
|---|---|---|
| 17 | $-CH_2-$ 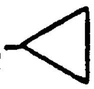 | ($CF_3CO_2D$): 0.2-1.5 (m, 5H, Cyclopropyl); 2.80 (s, 3H, $CH_3$); 3.48 und 3.67 (AB, J=18 Hz, 2H, $SCH_2$); 4.26 (d, 2H, $OCH_2$); 5.0-5.6 (m, 3H, $CH_2N$ und 6H); 6.10 (d, J=5Hz, 7-H); 7.26-7.55 (m, 3H, Thiazol-H und 2 Imidazol-H). |
| 18 | $-CH_2C{\equiv}CH$ | ($CF_3CO_2D$): 2.60 (t, 1H, ≡CH); 2.81 (s, 3H, $CH_3$); 3.45 und 3.65 (AB, 2H, $SCH_2$); 4.8-5.8 (m, 5H, $OCH_2$, $CH_2N$ und 6-H); 6.08 (d, J=5Hz, 7-H); 7.15-7.6 (m, 3H, Thiazol-H und 2 Imidazol-H). |
| 19 | $-CH_2CH{=}CH_2$ | ($CF_3CO_2D$): 2.81 (s, 3H, $CH_3$); 3.43 und 3.68 (AB, 2H, $SCH_2$); 4.7-4.9 (m, 2H, $\underline{CH_2}CH{=}CH_2$); 5.1-6.2 (m, 7H, $CH_2\underline{CH{=}CH_2}$, $CH_2N$ und 2 Laktam-H); 7.1-7.6 (m, 3H, Thiazol-H und 2 Imidazol-H). |
| 20 | $-CHF_2$ | ($CF_3CO_2D$): 2.80 (s, 3H, $CH_3$); 3.42 und 3.75 (AB, 2H, $SCH_2$); 4.9-5.7 (m, 3H, $CH_2N$ und 6-H); 6.11 (d, J=5Hz, 7-H); 6.72 (t, J=70Hz, 1H, $CHF_2$); 7.2-7.6 (m, 3H, Thiazol-H und 2 Imidazol-H). |

Beispiel 21:

Pivaloyloxymethyl-7-[2-(2-Aminothiazol-4 yl)-2-synmethoxyiminoaccetamido]-3-[(2-methyl-imidazol-1-yl)-methyl]-ceph-3-em-4-carboxylat

Ein Gemisch aus 954 mg (2 mmol) 7-[2-(2-Aminothiazol-4-yl)-2-syn-methoxyiminoacetamido]-3-[(2-methylimidazol-1-yl) methyl]-ceph-3-em-4-carbonsäure (Beispiel 1) und 200 mg (2 mmol) Kaliumhydrogencarbonat in 10 ml Wasser werden lyophilisiert, der Rückstand zweimal mit Toluol abgedampft und in 10 ml

trockenem N,N-Dimethylformamid (DMF) gelöst. Unter Rühren werden bei 5 ° C eine Lösung von 2 mmol Jodmethylpivalat in 0,5 ml DMF zugegeben, 2 Stunden gerührt, nochmal 0,5 ml Jodmethylpivalat-Lösung zugegeben und eine weitere Stunde gerührt. Nach Zugabe von 20 ml Wasser wird dreimal mit je 50 ml Essigsäureäthylester extrahiert, die organische Phase mit Natriumsulfat getrocknet und das Lösungsmittel i. Vak. entfernt. Der ölige Rückstand wird über eine "Lobar-C"-Kieselgelsäule mit Essigester:Isopropanol:Wasser (50:20:5) chromatographiert. Die Produktfraktionen werden abgedampft, der Rückstand mit Äther verrieben, wobei die Titelverbindung als amorpher Feststoff erhalten wird (140 mg).

$^1$H-NMR (DMSO-$d_6$): $\delta$ = 1.16 (s, 9H, $(CH_3)_3$); 2.25 (s, 3H, $CH_3$); 3.22 und 3.38 (AB, J = 18Hz, 2H, $SCH_2$); 3.83 (s, 3H, $OCH_3$); 4.88 (bs, AB, $CH_2N$); 5.19 (d, J = 5Hz, 6-H); 5.56 (dd, J = 5 und 9Hz, 7-H); 5.88 und 5.96 (AB, J = 7Hz, 2H, $\underline{CH_2}OCO$); 6.80 (s, 1Thiazol-H); 6.95 und 7.06 (je ein d, J = 2Hz, 2 Imidazol-H); 7.22 (bs, 2H, $NH_2$); 9.66 ppm (d; J = 9Hz, 1H, CONH).

Analog Beispiel 1, Verfahren b), Variante β) sowie den Beispielen 14 - 20 der Tabelle 2 werden die nachfolgend aufgeführten Beispiele erhalten, die der allgemeinen Formel I mit $R^1$ und $R^4$ = Wasserstoff entsprechen und worin $R^2$ und $R^3$ die in der Tabelle 3 angegebene Bedeutung haben.

## Tabelle 3

| Beispiel | $R^2$ | $R^3$ | $^1H$-NMR (Lösungsmittel) : δ (ppm) = |
|---|---|---|---|
| 22 | $-C_3H_7$ | 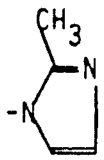 | ($CF_3CO_2D$) : 1,03 (t, 3H, $CH_3$); 1,86 (m, 2H, $CH_2$); 2,82 (s, 3H, $CH_3$); 3,43 und 3,60 (AB, 2H, $SCH_2$); 4,45 (t, 2H, $OCH_2$); 4,9 - 5,6 (m, 3H, 6-H und $CH_2N$); 6,10 (d, J = 5Hz, 1H, 7-H); 7,22 und 7,55 (m, 3H, Thiazol-H und 2 Imidazol-H) |
| 23 | $-(CH_2)_3CH_3$ | 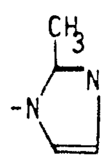 | ($CF_3CO_2D$) : 0,95 (t, 3H, $CH_3$); 1,15 - 1,95 (m, 4H, $CH_2CH_2$); 2,80 (s, 3H, $CH_3$); 3,40 und 3,73 (AB, 2H, $SCH_2$); 4,34 (t, 2H, $OCH_2$); 4,95 - 5,90 (m, 3H, 6-H und $CH_2N$); 6,13 (d, 1H, J = 5Hz, 7-H); 7,25 - 7,51 (m, 3H, Thiazol-H und 2 Imidazol-H) |
| 24 | $-CH_2-$ 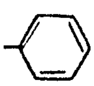 | 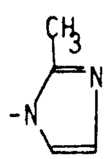 | ($CF_3CO_2D$) : 2,82 (s, 3H, $CH_3$); 3,26 und 3,43 (AB, 2H, $SCH_2$); 5,1 - 5,6 (m, 3H, 6-H und $CH_2N$); 5,45 (s, 2H, $OCH_2$); 6,03 (d, 1H, J = 5Hz, 7-H); 7,2 - 7,6 (m, 8H, Thiazol-H, 2 Imidazol-H, 5-Phenyl-H) |

Tabelle 3 (Fortsetzung)

| Beispiel | $R^2$ | $R^3$ | $^1$H-NMR (Lösungsmittel) : δ (ppm) = |
|---|---|---|---|
| 25 | 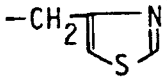 | 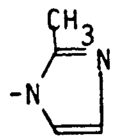 | ($CF_3CO_2D$) : 2,80 (s, 3H, $CH_3$); 3,45 und 3,65 (AB, 2H, $SCH_2$); 5,60 (s, 2H, $OCH_2$); 5,0 - 5,8 (m, 3H, 6-H) und $CH_2N$); 5,95 (d, 1H, J = 5Hz, 7-H); 7,25 (s, 1H, Thiazol-H); 7,6 - 7,9 (m, 2H, Imidazol-H); 8,20 und 9,85 (d, 2H, Thiazol-H) |
| 26 | 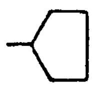 | 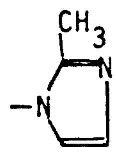 | ($DMSO-d_6$): 1,3 - 2,0 (m, 8H, Cyclopentyl); 2,33 (s, 3H, $CH_3$); 3,1 - 3,4 (m, 2H, $SCH_2$); 4,4 - 5,4 (m, 4H, 6-H, $CH_2N$,=OCH); 5,6 (dd, J = 5 und 8Hz, 7-H); 6,63 (s, 1H, Thiazol-H); 6,8 - 7,3 (m, 4H, 2 Imidazol-H, $NH_2$); 9,40 (d, J = 8Hz, 1H, CONH) |
| 27 | 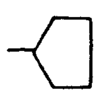 | 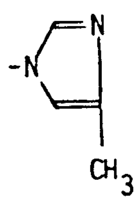 | ($CF3CO_2D$): 1,5 - 2,2 (m, 8H, Cyclopentyl); 2,43 (s, 3H, $CH_3$); 3,6 (bs, 2H, $SCH_2$); 4,9 - 5,6 (m, 4H, 6-H, $CH_2N$, =OCH); 6,03 (d, 1H, J= 5Hz, 7-H); 7,1 - 7,4 (m, 2H, Thiazol-H, Imidazol-H); 8,55 (bs, 1H, Imidazol-H) |

Analog Beispiel 1, Verfahren a) werden die Verbindungen der nachfolgend aufgeführten Beispiele als amorphe Feststoffe erhalten, die der allgemeinen Formel I mit $R^1$ und $R^4$ = Wasserstoff entsprechen und worin $R^2$ und $R^3$ die in der Tabelle 4 angegebene Bedeutung haben

Tabelle 4

| Beispiel | $R^2$ | $R^3$ | $^1$H-NMR (Lösungsmittel) : δ (ppm) = |
|---|---|---|---|
| 28 | $CH_3$ | 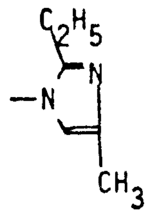 | (DMSO-$d_6$): 1,16 (t, 3H, $CH_2CH_3$); 2,12 (s, 3H, $CH_3$); 2,71 (q, 2H, $CH_2CH_3$); 2,84 und 3,04 (AB, J = 18Hz, 2H, $SCH_2$); 3,82 (s, 3H, $OCH_3$); 4,95 - 5,10 (m, 3H, $CH_2N$ und 6-H); 5,64 (dd, J = 5 und 8Hz, 7-H); 6,67 (s, Imidazol-H); 6,72 (s, Thiazol-H) 7,22 (bs, $NH_2$); 9,58 (d, J = 8Hz, CONH) |
| 29 | $CH_3$ | 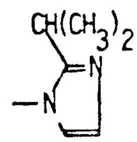 | (DMSO-$d_6$): 1,15 und 1,20 (je ein d, J = 7Hz, 2 x $CH_3$); 2,96 und 3,26 (AB, J = 18Hz, 2H, $SCH_2$); 3,16 (dq, $CH(CH_3)_2$; 3,81 (s, 3H, $OCH_3$); 4,91 (bs, 2H, $CH_2N$); 5,03 (d, J = 5Hz, 6-H); 5,64 (dd, J=5 und 8Hz, 7-H); 6,72 (s, Thiazol-H); 6,81 und 7,13 (je ein s, Imidazol-H); 7,22 (bs, $NH_2$); 9,57 (d, J = 8Hz, CONH) |
| 30 | $CH_3$ | 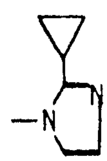 | (DMSO-$d_6$): 0,75 - 0,93 (m, 4 Cyclopropyl-H); 1,92 - 2.08 (m, 1 Cyclopropyl-H); 3,07 und 3,28 (AB, J = |

Tabelle 4 (Fortsetzung)

| Beispiel | $R^2$ | $R^3$ | $^1$H-NMR (Lösungsmittel): $\delta$ (ppm) = |
|---|---|---|---|
| | | | 18Hz, 2H, $SCH_2$); 3,82 (s, 3H, $OCH_3$); 4,94 und 5,04 (AB, J = 15Hz, 2H, $CH_2N$); 5,05 (d, J = 5Hz, 6-H); 5,66 (dd, J = 5 und 8Hz, 7-H); 6,69 (s, Imidazol-H); 6,71 (s, Thiazol-H); 7,13 (s, Imidazol-H); 7,22 (bs, $NH_2$); 9,57 (d, J = 8Hz, CONH) |
| 31 | $CH_3$ | 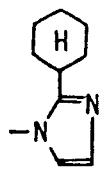 | ($DMSO-d_6$): 1,1 - 1,8 (m, 10 Cyclohexyl-H); 2,4 - 2,6 (m, 1 Cyclohexyl-H); 2,97 und 3,24 (AB, J = 18Hz, 2H, $SCH_2$); 3,82 (s, 3H, $OCH_3$); 4,89 und 4,94 (AB, J = 15Hz, 2H, $CH_2N$) 5,03 (d, J = 5Hz, 6-H); 5,65 (dd, J = 5 und 8Hz, 7-H); 6,72 (s, Thiazol-H); 6,80 und 7,12 (je 1 s, 2 Imidazol-H); 7,22 (bs, $NH_2$); 9,55 (d, J = 8Hz, CONH) |
| 32 | $CH_3$ | 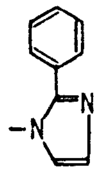 | ($DMSO-d_6$): 2,72 und 3,16 (AB, J = 18Hz, 2H, $SCH_2$); 3,82 (s, 3H, $OCH_3$); 4,82 und 5,04 (AB, J = 15Hz, 2H, $CH_2N$); 4,96 (d, J = 5Hz, 6-H); 5,49 (dd, J = 5 und 8Hz, 7-H); 6,71 (s, Thiazol-H); 6,98 (s, Imidazol-H); 7,21 (bs, $NH_2$); 7,4 - 7,64 (m, 6H, 1 Imidazol-H, 5 Phenyl-H); 9,53 (d, J = 8Hz, CONH) |

Tabelle 4 (Fortsetzung)

| Beispiel | $R^2$ | $R^3$ | $^1H$-NMR (Lösungsmittel) : δ (ppm) = |
|---|---|---|---|
| 33 | $CH_3$ | 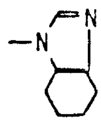 | (DMSO-$d_6$): 1,6 - 1,8 (bs, 4 Cyclohexen-H); 2,3 - 2,5 (bs, 4 Cyclohexen-H); 3,08 und 3,25 (AB, J = 18Hz, 2H, $SCH_2$); 3,82 (s, 3H, $OCH_3$); 4,81 und 4,88 (AB, J = 15Hz, 2H, $CH_2N$); 5,04 (d, J = 5Hz, 6-H); 5,65 (dd, J = 5 und 8Hz, 7-H); 6,72 (s, Thiazol-H); 7,22 (bs, $NH_2$); 7,64 (s, Imidazol-H); 9,58 (d, J = 8Hz, CONH) |
| 34 | $CH_3$ | 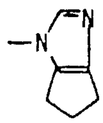 | (DMSO-$d_6$): 2,25 - 2,65 (m, 6 Cyclopenten-H); 3,10 und 3,31 (AB, J = 18Hz, 2H, $SCH_2$); 3,82 (s, 3H, $OCH_3$); 4,81 und 4,88 (AB, J = 15Hz, 2H, $CH_2H$); 5,08 (d, J = 5Hz, 6-H); 5,68 (dd, J = 5 und 8Hz, 7-H); 6,72 (s, Thiazol-H); 7,22 (bs, $NH_2$); 7,51 (s, Imidazol-H); 9,60 (d, J = 5Hz, CONH) |
| 35 | $CH_3$ | 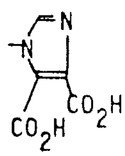 | (DMSO-$d_6$): 3,33 und 3,49 (AB, J = 18Hz, 2H, $SCH_2$); 3,82 (s, 3H, $OCH_3$); 3,94 und 4,22 (AB, J = 15Hz, 2H, $CH_2N$); 4,97 (d, J = 5Hz, 6-H); 5,58 (dd, J = 5 und 8Hz, 7-H); 6,74 (s, Thiazol-H); 7,21 (bs, $NH_2$); 7,62 (s, Imidazol-H); 9,51 (d, J = 8Hz, CONH); 12.9 (bs, COOH); 19,72 (bs, COOH) |

Tabelle 4 (Fortsetzung)

| Beispiel | $R^2$ | $R^3$ | $^1H$-NMR (Lösungsmittel) : $\delta$ (ppm) = |
|---|---|---|---|
| 36 | $CH_2CO_2H$ | 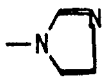 | ($DMSO-d_6$): 2,98 und 3,30 (AB, J = 18Hz, 2H, $SCH_2$); 4,34 (s, 2H, $OCH_2$); 4,74 und 4,92 (AB, J = 15Hz, 2H, $CH_2N$); 5,01 (d, J = 5Hz, 6-H); 5,63 (dd, J = 5 und 8Hz, 7-H); 6,81 (s, Thiazol-H); 6,85 , 7,28 und 7,73 (je 1 s, 3 Imidazol-H); 7,22 (bs, $NH_2$); 10,83 (d, J = 8Hz, CONH) |
| 37 | $CH_2CO_2H$ | 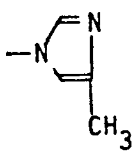 | ($CF_3CO_2D$): 2,50 (s, 3H, $CH_3$); 3,48 und 3,72 (AB, J = 18Hz, 2H, $SCH_2$); 5,11 (s, 2H, $OCH_2$); 5,23 und 5,59 (AB, J = 15Hz, 2H, $CH_2N$); 5,41 (d, J = 5Hz, 6-H); 6,14 (d, J = 5Hz, 7-H); 7,30 (s, Imidazol-H); 7,46 (s, Thiazol-H); 8,77 (s, Imidazol-H) |
| 38 | $C(CH_3)_2CO_2H$ | 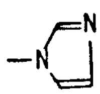 | ($DMSO-d_6$): 1,43 (bs, 6H, $CH_3$); 3,00 und 3,33 (AB, J = 18Hz, 2H, $SCH_2$); 4,71 und 4,94 (AB, J = 15Hz, 2H, $CH_2N$) 5,05 (d, J = 5Hz, 6-H); 5,68 (dd, J = 5 und 8Hz, 7-H); 6,71 (s, Thiazol-H); 6,85 (s, Imidazol-H); 7,28 (bs, 3H, $NH_2$ und 1 Imidazol-H); 7,72 (s, Imidazol-H); 9,58 (d, J = 8Hz, CONH) |
| 39 | $C(CH_3)_2CO_2H$ | 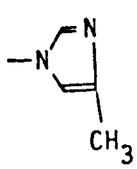 | ($DMSO-d_6$) : 1,88 und 1,95 (je ein s, 2 x $CH_3$); 2,11 (s, 3H, $CH_3$); 2,95 und 3,03 (AB, J = 18Hz, 2H, $SCH_2$); 4,71 und 5,02 (AB, J = 15Hz, 2H, $CH_2N$); 4,96 (d, J = 5Hz, 6-H); 5,61 (dd, J = 5 und 8Hz, 7-H); 6,58 (s, |

Tabelle 4 (Fortsetzung)

| Beispiel | $R^2$ | $R^3$ | $^1$H-NMR (Lösungsmittel): δ (ppm) = |
|---|---|---|---|
| | | | Imidazol-H); 6,70 (s, Thiazol-H); 7,21 (bs, $NH_2$); 7,61 (s, Imidazol-H) |
| 40 | 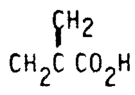 | 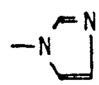 | (DMSO-$d_6$): 2,91 und 3,32 (AB, J = 18Hz, 2H, $SCH_2$); 4,63 und 4,93 (AB, J = 15Hz, 2H, $CH_2N$); 4,70 (s, 2H, $OCH_2$); 5,01 (d, J = 5Hz, 6-H); 5,60 (dd, J = 5 und 8Hz, 7-H); 5,80 und 6,12 (je 1 s, $=CH_2$); 6,72 (s, Thiazol-H); 6,83 , 7,28 und 7,71 (je 1 s, 3 Imidazol-H); 7,21 (bs, $NH_2$); 9,61 (d, J = 8Hz, CONH) |
| 41 | 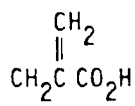 | 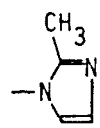 | (DMSO-$d_6$): 2,26 (s, 3H, $CH_3$); 2,93 und 3,18 (AB, J = 18Hz, 2H, $SCH_2$); 4,73 (s, 2H, $OCH_3$); 4,80 und 4,89 (AB, J = 15Hz, 2H, $CH_2N$); 5,00 (d, J = 5Hz, 6-H); 5,58 (dd, J = 5 und 8Hz, 7-H); 5,63 und 6,00 (je 1 bs, 2H, $=CH_2$); 6,66 und 7,16 (je 1 s, 2 Imidazol-H); 6,71 (s, Thiazol-H); 7,24 (bs, $NH_2$); 9,61 (d, J = 8Hz, CONH) |
| 42 | 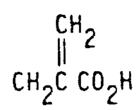 | 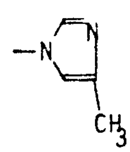 | (DMSO-$d_6$): 2,11 (s, 3H, $CH_3$); 2,94 und 3,12 (AB, J = 18Hz, 2H, $SCH_2$); 4,72 (s, 2H, $OCH_2$); 4,80 und 4,90 (AB, J = 15Hz, 2H, $CH_2N$); 5,00 (d, J = 5Hz, 6-H); 5,60 (dd, J = 5 und |

Tabelle 4 (Fortsetzung)

| Beispiel | $R^2$ | $R^3$ | $^1H$-NMR (Lösungsmittel) : δ (ppm) = |
|---|---|---|---|
| | | | 8Hz, 7-H); 5,80 und 6,12 (je 1 s, 2H, $=CH_2$); 6,58 und 7,63 (je 1 s, 2 Imidazol-H); 6,72 (s, Thiazol-H); 7,22 (bs, $NH_2$); 9,62 (d, J = 8Hz, CONH) |
| 43 | 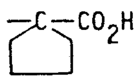 | 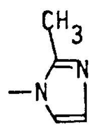 | ($CF_3CO_2D$): 1,8 - 2,7 (m, 8H, Cyclopentyl); 2,81 (s, 3H, $CH_3$); 3,46 und 3,66 (AB, J = 18Hz, 2H, $SCH_2$); 5,0 - 5,55 (m, 3H, 6-H und $CH_2N$); 6,10 (d, J = 5Hz, 7-H); 7,24 - 7,53 (m, 3H, Thiazol-H und 2 Imidazol-H) |
| 44 | $CH_3$ | 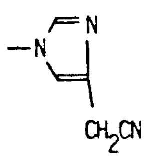 | (DMSO-$d_6$): 3,22 und 3,43 (AB, 2H, $SCH_2$), 3,80 (s, 2H, $CH_2CN$); 3,82 (s, 3H, $OCH_3$); 4,78 und 4,94 (AB, 2H, $CH_2N$); 5,02 (d, J = 5Hz, 6-H); 5,57 (dd, J = 5 und 8Hz, 7-H); 6,74 (s, Thiazol-H); 6,86 und 7,74 (je 1 s, 2 Imidazol-H); 7,21 (bs, $NH_2$); 9,50 (d, J = 8Hz, CONH) |
| 45 | $CH_3$ | 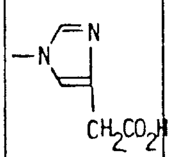 | (DMSO-$d_6$): 3,25 und 3,36 (AB, 2H, $SCH_2$); 3,80 (s, 2H, $CH_2CO_2H$); 3,82 (s, 3H, $OCH_3$); 4,81 und 4,94 (AB, 2H, $CH_2N$); 5,02 (d, J = 5Hz, 6-H); 5,55 (dd, J= 5 und 8Hz, 7-H), 6,74 (s, Thiazol-H); 6,86 und 7,74 (je 1 s, 2 Imidazol-H); 7,20 (bs, $NH_2$); 9,48 (d, J = 8Hz, CONH); |

| Beispiel | $R^2$ | $R^3$ | $^1H$-NMR (Lösungsmittel): δ (ppm)= |
|---|---|---|---|
| 46 | $CH_3$ | 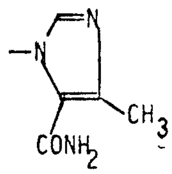 | ($DMSO-d_6$): 2,30 (s, 3H, $CH_3$); 3,18 und 3,48 (AB, $SCH_2$); 3,81 (s, 3H, $OCH_3$); 4,85-5,05 (m, 3H, 6-H und $CH_2N$); 5,58 (dd, J = 5 und 8Hz, 7-H); 6,72 (s, Thiazol-H); 7,20 (bs, $NH_2$); 7,62 (s, Imidazol-H); 9,48 (d, J = 8Hz, NH) |
| 47 | $CH_3$ | 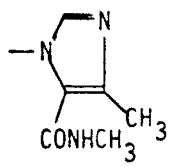 | ($DMSO-d_6$): 2,42 (s, 3H, $CH_3$); 2,70 (d, J = 7Hz, 2 x $CH_3$); 3,22 und 3,42 (AB, $SCH_2$); 3,81 (s, 3H, $OCH_3$); 4,82 - 5,03 (m, 3H, 6-H und $CH_2N$); 5,51 (dd, J = 5 und 8Hz, 7-H); 6,71 (s, Thiazol-H); 7,18 (bs, $NH_2$); 7,72 (s, Imidazol-H); 9,48 (d, J = 8Hz, NH) |
| 48 | $CH_3$ | 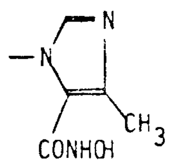 | ($DMSO-d_6$): 2,40 (s, 3H, $CH_3$); 3,21 und 3,46 (AB, $SCH_2$); 3,81 (s, 3H, $OCH_3$); 4,8-5,1 (m, 3H, 6-H und $CH_2N$); 5,61 (dd, J = 5 und 8Hz, 7-H); 6,71 (s, Thiazol-H); 7,21 (bs, $NH_2$); 7,68 (s, Imidazol-H); 9,50 (d, NH) |
| 49 | $CH_3$ | 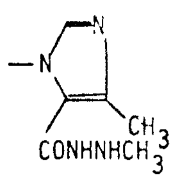 | ($CF_3CO_2D$): 2,30 (s, 3H, $CH_3$); 2,80 (s, 3H, $CH_3$); 3,88 und 3,99 (AB, J= 18Hz, 2H, $SCH_2$); 4,26 (s, 3H, $OCH_3$); 4,65 und |

| Beispiel | $R^2$ | $R^3$ | $^1$H-NMR (Lösungsmittel): δ (ppm)= |
|---|---|---|---|
| | | | 4,92 (AB, J = 15Hz, 2H, $CH_2N$); 5,36 (d, J = 5Hz, 6-H); 6,10 (d, J = 5Hz, 7-H); 7,44 (s, Thiazol-H); 8,83 (s, Imidazol-H) |
| 50 | $C_2H_5$ | 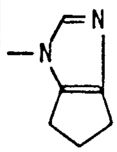 | ($DMSO-d_6$): 1,20 (t, 3H, $CH_2CH_3$); 2,25 - 2,68 (m, 6 Cyclopenten-H); 3,02 und 3,28 (AB, J = 18Hz, 2H, $SCH_2$); 3,95 (q, 2H, $CH_2CH_3$); 4,88 (AB, 2H, $CH_2N$); 5.04 (d, J = 5Hz, 6-H); 5,61 (dd, J = 5 und 8Hz, 7-H); 6,70 (s, Thiazol-H); 7,22 (bs, $NH_2$); 7,49 (s, Imidazol-H); 9,51 (d, J = 8Hz, NH) |
| 51 | $CH_2C \equiv CH$ | 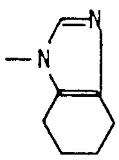 | ($DMSO-d_6$): 1,6 - 1,8 (bs, 4 Cyclohexen-H); 2,3-2,6 (m, 5H, CH und 4 Cyclohexen-H); 3,02 und 3,20 (AB, J = 18Hz, 2H, $SCH_2$); 4,57 (bs, 2H, $OCH_2$); 4,75 - 4,90 (AB, 2H, $CH_2N$); 5,03 (d, J = 5Hz, 6-H); 5,61 (dd, J = 5 und 8Hz, 7-H); 6,76 (s, Thiazol-H); 7,24 (bs, $NH_2$); 7,58 (s, Imidazol-H); 9,63 (d, J = 8Hz, CONH) |
| 52 | $C_3H_7$ | 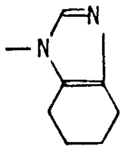 | ($CF_3CO_2D$): 1,1 (t, 3H, $CH_3$); 1,7-2,3 (m, 6H, $CH_2$ und 4 Cyclohexen-H); 2,6-2,9 (bs, 4 Cyclohexen-H); 3,45 und 3,76 (AB, 2H, $SCH_2$); 4,50 (t, 2H, $OCH_2$); |

| Beispiel | $R^2$ | $R^3$ | $^1$H-NMR (Lösungsmittel): δ (ppm)= |
|---|---|---|---|
| | | | 4,90 und 5,85 (m, 3H, 6-H und $CH_2N$); 6,13 (d, 1H, 7-H); 7,50 (s, Thiazol-H); 8,67 (s, Imidazol-H) |
| 53 | 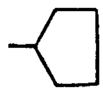 | 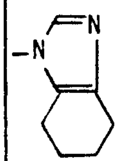 | ($CF_3COOD$): 1,6 - 2,4 (m, 12H, 8 Cyclopentyl-H und 4 Cyclohexen-H); 2,6-2,95 (m, 4H, 4 Cyclohexen-H); 3,50 und 3,68 (AB, 2H, $SCH_2$); 5.05 - 5,55 (m, 4H,Laktam-H, $CH_2N$ und OCH); 6,14 (d, 1H, 7-H); 7,50 (s, Thiazol-H); 8,66 (s, Imidazol-H) |
| 54 | $C_3H_7$ | 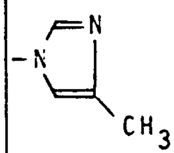 | ($CF_3COOD$):1.04 (t, 3H, $CH_3$); 1,83 (m, 2H, $CH_2$); 2,5 (s, 3H, $CH_3$); 3,62 (bs, 2H, $SCH_2$); 4,50 )t, 2H, $OCH_2$); 5.15 - 5,60 (m, 3H, 6-H und $CH_2N$); 6,14 (d, 1H, 7-H); 7,23 (bs, Imidazol-H); 7,50 (s, Thiazol-H) |
| 55 | 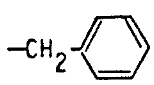 | 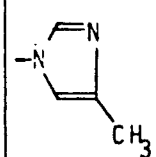 | ($CF_3COOD$): 2,50 (s, 3H, $CH_3$); 3,28 und 3,46 (AB, 2H, $SCH_2$); 5,1-5,65 (m, 5H, Laktam-H, $CH_2N$ und $OCH_2$); 6,08 (d, 1H, 7-H); 7,21 (bs, Imidazol-H); 7,30-7,60 (m, 6H, 5 Benzyl-H und 1 Thiazol-H); 8,59 (bs, Imidazol-H) |

| Beispiel | $R^2$ | $R^3$ | $^1H$-NMR (Lösungsmittel): δ (ppm)= |
|---|---|---|---|
| 56 | -$CH_2$- 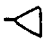 | 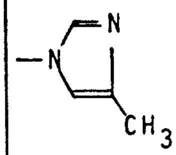 | ($CF_3COOD$): 0,2-1,5 (m, 5H, Cyclopropyl-H); 2,48 (s, 3H, $CH_3$); 3,60 (bs, 2H, $SCH_2$); 4,40 (d, 2H, $OCH_2$); 5,05 - 5.65 (m, 3H, 6-H und $CH_2N$); 6,13 (d, 1H, 7-H); 7,20 (bs, 1H, Imidazol-H); 7,50 (s, Thiazol-H); 8,78 (s, Imidazol-H) |
| 57 | $CH_3$ | 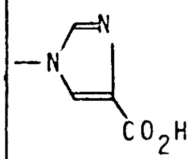 | ($CF_3COOD$): 3,70 (bs, 2H, $SCH_2$); 4,30 (s, 3H, $OCH_3$); 5,15 - 5,80 (m, 3H, 6-H und $CH_2N$); 6,15 (d, 7-H); 7,50 (s, Thiazol-H); 8,30 und 9,03 (bs, 2 Imidazol-H) |
| 58 | $CH_3$ | 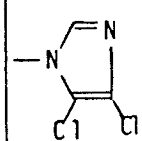 | ($CF_3COOD$): 3,55 und 3,78 (AB, 2H, $SCH_2$); 4,25 (s, 3H, $OCH_3$); 5.05 - 5,50 (m, 3H, 6-H und $CH_2N$); 6,15 (d, 1H, 7-H); 7,50 (s, Thiazol-H); 8,48 (s, Imidazol-H) |
| 59 | $CH_3$ | 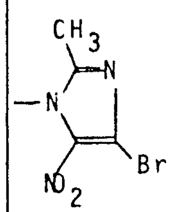 | ($CF_3COOD$): 2,83 (s, 3H, $CH_3$); 3,60 (bs, 2H, $SCH_2$); 4,25 (s, 3H, $OCH_3$); 5.05 - 5,60 (m, 3H, Laktam-H und $CH_2N$); 6,13 (d, 1H, 7-H); 7,51 (s, 1H, Thiazol-H) |

| Beispiel | $R^2$ | $R^3$ | $^1$H-NMR (Lösungsmittel): δ (ppm)= |
|---|---|---|---|
| 60 | $CH_3$ | 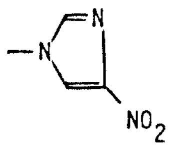 | ($CF_3COOD$): 3,75 (bs, 2H, $SCH_2$); 4,30 (s, 3H, $OCH_3$); 5,0 - 5,5 (m, 3H, Laktam-H und $CH_2N$); 6,15 (d, 1H, 7-H); 7,50 (s, Thiazol-H); 7,55 und 8,50 (bs, Imidazol-H) |
| 61 | $CH_3$ | 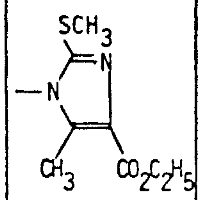 | ($CF_3COOD$): 1,48 (t, 3H, $CH_3$); 2,75 (s, 3H, $CH_3$); 2,95 (s, 3H, $SCH_3$); 3,62 (bs, 2H, $SCH_2$); 3.83 (q, 2H, $OCH_2$); 4,28 (s, 3H, $OCH_3$); 5,0 - 5,55 (m, 3H, 6-H und $CH_2N$); 6,08 (d, 1H, 7-H); 7,50 (s, Thiazol-H) |
| 62 | $CH_3$ | 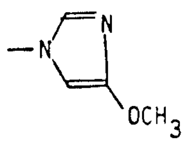 | ($CF_3COOD$): 3,46 und 3,70 (AB, 2H, $SCH_2$); 4,15 (s, 3H, $OCH_3$); 4,25 (s, 3H, $NOCH_3$); 5,00 - 5,65 (m, 3H, 6-H und $CH_2N$); 6,13 (d, 1H, 7-H); 7,15 (bs, Imidazol-H); 7,50 (s, Thiazol-H) 8,72 (s, Imidazol-H) |
| 63 | $CH_3$ ($R^1$ = Cl) | 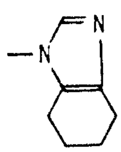 | (DMSO-$d_6$): 1,6-1,8 (bs, 4-Cyclohexen-H); 2,35-2,6 (bs, 4 Cyclohexen-H); 2.98 und 3,18 (AB, J = 18Hz, 2H, $SCH_2$); 3,82 (s, 3H, $OCH_3$); 4,78 und 4,85 (AB, J = 15Hz, 2H, $CH_2N$); 4,98 (d, J = 5Hz, 6-H); 5,60 (dd, J = 5 und 8Hz, 7-H); 7,38 (bs, $NH_2$); 7,56 (s, Imidazol-H); 9,48 (d, J = 8Hz, CONH) |

| Beispiel | $R^2$ | $R^3$ | $^1H$-NMR (Lösungsmittel): δ (ppm)= |
|---|---|---|---|
| 64 | $CH_2COOH$ | 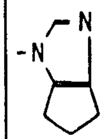 | ($DMSO-d_6$): 2,25-2,65 (m, 6 Cyclopenten-H); 2,98 und 3,18 (AB, J = 18Hz, 2H, $SCH_2$); 4,22 (s, 2H, $OCH_2$); 4,71 und 4,93 (AB, J = 15Hz, 2H, $CH_2N$); 4,95 (d, J = 5Hz, 6-H); 5,57 (dd, J = 5 und 8Hz, 7-H); 6,81 (s, Thiazol-H); 7,16 (bs, $NH_2$); 7,47 (s, Imidazol-H); 10,90 (d, J =8Hz, CONH) |
| 65 | $C(CH_3)_2CO_2H$ | 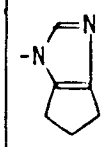 | ($DMSO-d_6$): 1,45 (bs, 6H, $CH_3$); 2,20 - 2,60 (m, 6 Cyclopenten-H) 3,00 und 3,28 (AB, J=18Hz, 2H, $SCH_2$); 4,70 und 4,92 (AB, J = 15Hz, 2H, $CH_2N$); 5,01 (d, J = 5Hz, 6-H); 5,62 (dd, J = 5 und 8Hz, 7-H); 6,82 (s, Thiazol-H) 7,15 (bs, $NH_2$); 7,49 (s, Imidazol-H); 10,92 (d, J 8Hz, CONH) |
| 66 | $CH_2CO_2H$ | 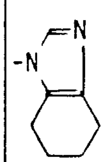 | ($DMSO-d_6$): 1,6-1,8 (bs, 4 Cyclohexen-H); 2,3-2,55 (bs, 4 Cyclohexen-H); 3,02 und 3,15 (AB, J = 18Hz, 2H, $SCH_2$); 4,34 (s, 2H, $OCH_2$); 4,72 und 4,94 (AB, J = 15Hz, 2H, $CH_2N$); 5,01 (d, J = 5Hz, 6-H); 5,62 (dd, J = 5 und 8Hz, 7-H); 6,82 (s, Thiazol-H) 7,20 (bs, $NH_2$); 7,57 (s, Imidazol-H); 11,10 (d, J = 8Hz, CONH) |

| Beispiel | $R^2$ | $R^3$ | $^1H$-NMR (Lösungsmittel): δ (ppm)= |
|---|---|---|---|
| 67 | $C(CH_3)_2CO_2H$ | 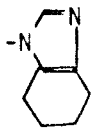 | ($DMSO-d_6$): 1,45 (bs, 6H, $CH_3$); 1,6 - 1,8 (bs, 4 Cyclohexen-H), 2,3-2,6 (bs, 4 Cyclohexen-H); 3,01 und 3,19 (AB, J = 18Hz, 2H, $SCH_2$); 4,72 und 4,94 (AB, J = 15Hz, 2H, $CH_2N$); 5,00 (d, J = 5Hz, 6-H); 5,60 (dd, J = 5 und 8Hz, 7-H); 6,81 (s, Thiazol-H); 7,20 (bs, $NH_2$); 7,55 (s, Imidazol-H); 11,05 (d, J = 8Hz, CONH) |
| 68 | $C_2H_5$ | 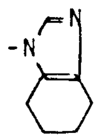 | ($DMSO-d_6$):1,22 (t, 3H, $CH_2\underline{CH_3}$) 1,6-1,8 (bs, 4 Cyclohexen-H) ; 2,3-2,5 (m, 4 Cyclohexen-H); 2,97 und 3,16 (AB, J = 18Hz, 2H, $SCH_2$); 4.06 (q, 2H, $\underline{CH_2}CH_3$); 4,77 und 4,86 (AB, J = 15Hz, 2H, $CH_2N$); 5,00 (d, J = 5Hz, 6-H); 5,58 (dd, J = 5 und 8Hz, 7-H); 6,70 (s, Thiazol-H); 7,20 (bs, $NH_2$); 7,52 (s, Imidazol-H); 9,48 (d, J = 8Hz, CONH) |
| 69 | $C_2H_5$ | 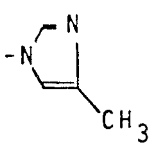 | ($DMSO-d_6$): 1,20 (s, 3H, $CH_2\underline{CH_3}$) 2,11 (s, 3H, $CH_3$); 2,92 und 3,13 (d, J = 18Hz, 2H, $SCH_2$); 4,06 (q, 2H, $\underline{CH_2}CH_3$); 4,78 und 4,89 (AB, J = 15Hz, 2H, $CH_2N$); 4,98 (d, J = 5Hz, 6-H); 5,57 (dd, J = 5 und 8Hz, 7-H); 6,56 (s, Imidazol-H); 6,69 (s, Thiazol-H); 7,18 (bs, $NH_2$); 7,62 (s, Imidazol-H); 9,47 (d, J = 8Hz, CONH) |

| Beispiel | $R^2$ | $R^3$ | $^1$H-NMR (Lösungsmittel): δ (ppm) = |
|---|---|---|---|
| 70 | 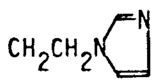 | 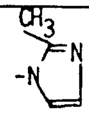 | ($DMSO-d_6$): 2,27 (s, 3H, $CH_3$); 2,92 und 3,21 (d, J = 18Hz, 2H, $SCH_2$); 4,23 (bs, 4H, $OCH_2CH_2$); 4,81 und 4,88 (d, J = 15Hz, 2H, $CH_2N$); 5,01 (d, J = 5Hz, 6-H); 5,58 (dd, J = 5 und 8Hz, 7-H); 6,68 (s, Imidazol-H); 6,76 (s, Thiazol-H); 6,84, 7,16 7,18 und 7,60 (je 1 s, 4 Imidazol-H); 7,24 (bs, $NH_2$); 9,59 (d, J = 8Hz, CONH) |
| 71 | $CH_3$ | 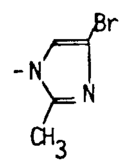 | ($CF_3COOD$): 2,90 (s, 3H, $CH_3$); 3,25 und 3,48 (AB, 2H, $SCH_2$); 4,25 (s, 3H, $OCH_3$); 5,05-5,65 (m, 3H, Laktam-H und $CH_2N$); 6,12 (d, 1H, J = 5Hz, Laktam-H); 7,48 (s, 1H, Thiazol-H); 7,52 (s, 1H, Imidazol-H) |
| 72 | $CH_3$ | 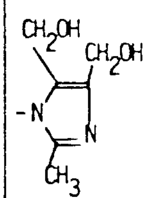 | ($CF_3COOD$): 2,84 (s, 3H, $CH_3$); 3,45 (bs, 2H, $SCH_2$); 4,27 (s, 3H, $OCH_3$); 5,07 (s, 4H, $CH_2OH$), 5,20 - 5,85 (m, 3H, Laktam-H und $CH_2N$); 6,05 (d, J = 5Hz, 1H, Laktam-H); 7,48 (s, 1H, Thiazol-H) |
| 73 | 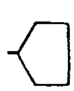 | 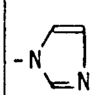 | ($CF_3COOD$): 1,5-2,2 (m, 8H, Cyclopentyl); 3,50 und 3,90 (AB, 2H, $SCH_2$); 5,0-5,9 (m, 4H, Laktam-H, $CH_2N$, OCH); 6,16 (d, |

| Beispiel | $R^2$ | $R^3$ | $^1$H-NMR (Lösungsmittel) : δ (ppm)= |
|---|---|---|---|
| | | | J = 5Hz, 1H, Laktam-H); 7,50 (s, 1H, Thiazol-H); 7,55; 7,70 und 9,00 (je ein bs, 3 Imidazol-H) |
| 74 | $CH_2CONH_2$ | 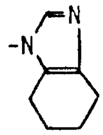 | (DMSO-$d_6$): 1,6-1,8 (bs, 4 Cyclohexen-H); 2,3 - 2,6 (bs, 4 Cyclohexen-H); 2,96 und 3,21 (AB, J = 18Hz, 2H, $SCH_2$); 4,39 (s, 2H, $CH_2CONH_2$); 4,76 und 4,88 (AB, J = 15Hz, 2H, $CH_2N$); 5,02 (d, J = 5Hz, 6-H); 5,63 (dd, J = 5 und 8Hz, 7-H); 6,82 (s, Thiazol-H); 7,10 (s, 1H, $NH_2CO$); 7,29 (s, 2H $NH_2$); 7,48 (s, 1H, $NH_2CO$); 7,52 (s, 1 Imidazol-H); 9,73 (d, J = 8Hz, CONH) |
| 75 | $CH_2CONHCH_3$ | 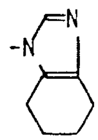 | (DMSO-$d_6$): 1,6-1,75 (bs, 4 Cyclohexen-H); 2,45-2,5 (bs, 4 Cyclohexen-H); 2,64 und 2,66 (2 s, 3H, $NHCH_3$); 2,98 und 3,18 (AB, J = 18Hz, 2H, $SCH_2$); 4,43 (s, 2H, $CH_2CONHCH_3$); 5,26 und 5,88 (AB, J = 15Hz, 2H, $CH_2N$); 5,02 (d, J = 5Hz, 6-H); 5,61 (dd, J = 5 und 8Hz, 7-H); 6,83 (s, Thiazol-H); 7,30 (s, 2H, $NH_2$); 7,42 und 7,44 (2 s, 1H, $NHCH_3$); 7,49 (s, 1 Imidazol-H); 9,68 (d, J = 8Hz, CONH) |

Beispiel 76 :

7-[2-(2-Aminothiazol-4-yl)-2-syn-methoxyimino-acetamido ] -3- [(2-methylimidazol-1-yl)methyl ]-ceph-3-em-4-carbonsäure

Verfahren b), Variante β

1. Tert. Butyl-7-Amino-3-[(2-methylimidazol-1-yl)methyl] - ceph-3-em-4-carboxylat

1,77 g (5,4 mmol) 7-Aminocephalosporansäure-tert. butylester und 5 ml (18,7 mmol) BSTFA in 12 ml Methylendichlorid werden 1 Stunde zum Sieden erhitzt. Nach Kühlen werden 2 ml (15 mmol) Trimethyljodsilan zugegeben und 20 Minuten bei Raumtemperatur belassen. Es wird eingeengt, der ölige Rückstand in 10 ml Acetonitril gelöst und eine Lösung von 524 mg (6,4 mmol) 2-Methyl-imidazol in 5,2 ml Acetonitril und 2,6 ml BSTFA zugegeben. Es wird 3 Stunden bei Raumtemperatur belassen. Nach Zugabe von 0,6 ml Wasser bildet sich eine geringe Menge eines dunklen Niederschlages, der abgesaugt (140 mg) und verworfen wird. Die Mutterlauge wird eingeengt, der Rückstand über eine Kieselgelsäule mit Methylendichlorid/Methanol/wässr. Ammoniak (90 : 10 : 1) chromatographiert. Die Fraktionen 3-5 (130 ml) werden abgedampft und es verbleiben 310 mg der Titelverbindung als amorpher, bräunlicher Feststoff.

$^1$H-NMR (DMSO-$d_6$): $\delta$ = 1,50 (s, 9H, $(CH_3)_3$); 2,29 (s, 3H, Im.-$CH_3$); 3,18 und 3,27 (AB, J = 18Hz, 2H, $SCH_2$); 5,78 und 5,88 (AB, J = 15Hz, $CH_2N$); 4,80 und 5,01 (je 1 d, J = 5Hz, 2 Lactam-H); 6,90 und 7,12 ppm (2 s, je 1 Imidazol-H)

2. Tert. Butyl-7-[ 2-(2-Aminothiazol-4-yl)-2-syn-methoxyiminoacetamido]-3-[(2-methylimidazol-1-yl)methyl ]-ceph-3-em-4-carboxylat

Zur Lösung von 0,5 mmol 2-(2-Aminothiazol-4-yl)-2-syn-methoxyiminoessigsäure-Hydroxybenztriazol-Aktivester, hergestellt aus 100 mg (0,5 mmol) Säure, 90 mg Hydroxybenztriazol und 110 mg Dicyclohexylcarbodiimid in 2,5 ml N,N-Dimethylformamid (DMF), wird eine Lösung von 190 mg (0,54 mmol) Produkt von Beispiel 76/1 in 2 ml DMF zugegeben. Nach 4 Stunden bei Raumtemperatur wird vom Dicyclohexylharnstoff abgesaugt, die Lösung mit 150 ml Äthylacetat verdünnt und zweimal mit wässr. Natriumbicarbonatlösung sowie zweimal mit Wasser gewaschen. Nach Trocknen mit $MgSO_4$ wird das Lösungsmittel im Vakuum entfernt, der z.T. feste Rückstand mit Äther digeriert. Der ungelöste Feststoff wird abgesaugt, mit Äther gewaschen und getrocknet. Ausbeute: 150 mg

$^1$H-NMR (DMSO-$d_6$): $\delta$ = 1,48 (s, 9H, $(CH_3)_3$); 2,28 (s, 3H, $CH_3$); 3,28 und 3,39 (AB, J = 18Hz, 2H, $SCH_2$); 3,84 (s, 3H, $OCH_3$); 4,82 und 4,93 (AB, J = 15Hz, 2H, $CH_2N$); 5,28 (d, J = 5Hz, 6-H); 5,60 (dd, J= 5 und 8Hz, 7-H); 6,74 (s, Thiazol-H); 6,86 (s, Imidazol-H); 7,21 (bs, 3H, $NH_2$ und 1 Imidazol-H); 9,60 ppm (d, J = 8Hz, CONH)

3. Herstellung der Titelverbindung

30 mg der Verbindung von Beispiel 76/2 werden in 3 ml Trifluoressigsäure gelöst. Nach 30 Minuten wird i. Vak. abgedampft, der Rückstand mehrmals mit Äther verrieben. Man erhält 25 mg eines farblosen Feststoffs der in allen Eigenschaften (RF - Wert, NMR-Spektrum) mit der Verbindung von Beispiel 1 identisch ist.

**Patentansprüche**

**Patentansprüche für folgende Vertragsstaaten : BE, CH, DE, FR, GB, IT, LI, LU, NL, SE**

1. Cephalosporinderivate der allgemeinen Formel I

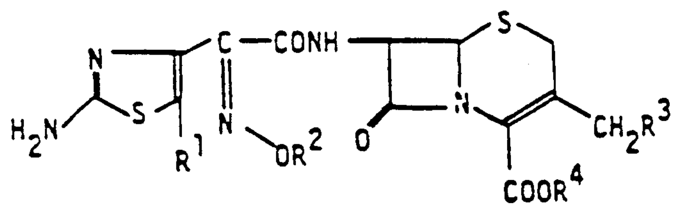

(I)

und deren physiologisch verträgliche Säureadditionssalze, worin

$R^1$ Wasserstoff oder Fluor, Chlor oder Brom

$R^2$ Wasserstoff,

$C_1$-$C_6$-Alkyl, das ein- oder mehrfach, gleich oder verschieden substituiert sein kann durch Fluor, Phenyl, 2- oder 3- oder 4-Tolyl, 2- oder 3- oder 4-Chlorphenyl, 1,3-Thiazol-4-yl, Imidazol-1-yl, $C_1$-$C_4$-Alkylthio, $C_1$-$C_4$-Alkyloxy, Nitril oder Carbamoyl, worin die Aminogruppe ein- oder zweifach, gleich oder verschieden substituiert sein kann durch $C_1$-$C_6$-Alkyl, Hydroxy-($C_1$-$C_2$)-alkyl, Hydroxy oder Methoxy;

$C_2$-$C_6$-Alkenyl, das gegebenenfalls ein- oder mehrfach, gleich oder verschieden substituiert sein kann durch Chlor oder Brom,

$C_2$-$C_6$-Alkinyl, das gegebenenfalls substituiert sein kann durch Halogen,

$C_3$-$C_7$-Cycloalkyl, das gegebenenfalls substituiert sein kann durch Halogen,

$C_4$-$C_7$-Cycloalkenyl,

$C_3$-$C_7$-Cycloalkylmethyl,

die Gruppen $-CH_2=CH-R^7$ oder

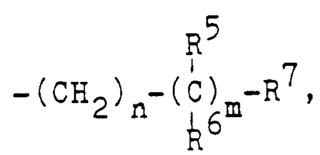

worin

m and n jeweils für 0 oder 1 steht,

$R^5$ und $R^6$ gleich oder verschieden sein können und Wasserstoff, Phenyl oder eine $C_1$-$C_{11}$-Alkylgruppe bedeuten, oder zusammen mit dem Kohlenstoff, an das sie gebunden sind, eine Methylen- oder eine $C_3$-$C_7$-Cycloalkylidengruppe bilden,

$R^7$ eine HOOC- oder $C_1$-$C_4$-Alkyl-OOC-Gruppe

$R^3$ einen Imidazol-1-yl-Rest

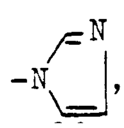

der ein- oder mehrfach, gleich oder verschieden substituiert sein kann durch $C_1$-$C_6$-Alkyl, das ein- oder mehrfach substituiert sein kann durch Hydroxy, Acetoxy, Carbamoyloxy, Chlor, Carboxy, $C_1$-$C_6$-Alkyloxycarbonyl, Formyl, $C_1$-$C_6$-Alkylcarbonyl, Cyano, Carbamoyl, $C_1$-$C_6$-Alkyloxy und wobei 2 benachbarte Alkylgruppen auch zu einem gegebenenfalls durch $C_1$-$C_6$-Alkyl, Halogen, Hydroxy, Oxo, Carboxy, Carbamoyl substituierten Di- bis Decamethylen-Ring geschlossen sein können, in dem ein C-Atom durch eine Sauerstoff- oder Schwefelatom

ersetzt sein kann und auch eine oder zwei Doppelbindungen enthalten sein können,
durch $C_2$-$C_6$-Alkenyl, das durch Hydroxy substituiert sein kann,
durch $C_2$-$C_6$-Alkinyl,
durch $C_3$-$C_6$-Cycloalkyl
durch $C_3$-$C_6$-Cycloalkylmethyl
durch Phenyl oder Benzyl, die durch $C_1$-$C_4$-Alkyl, Fluor, Chlor, Hydroxy, $C_1$-$C_4$-Alkoxy substituiert sein können,
durch $C_1$-$C_6$-Alkoxy oder durch $C_1$-$C_6$-Alkylthio,
durch Fluor, Chlor, Brom, Jod, Trifluormethyl, Cyano, Hydroxy oder Mercapto,
durch Carboxy, $C_1$-$C_6$-Alkoxycarbonyl oder Carbamoyl, das am Stickstoff ein- oder zweimal substituiert sein kann durch $C_1$-$C_6$-Alkyl, Hydroxy oder Methoxy,
durch Carbazoyl, das am Stickstoff ein- oder zweimal substituiert sein kann durch $C_1$-$C_4$-Alkyl,
durch Nitro, Amino oder Di-($C_1$-$C_2$)-alkylamino
durch Formyl, $C_1$-$C_4$-Alkylcarbonyl oder Benzoyl,

$R^4$ Wasserstoff, $C_1$-$C_6$-Alkyl, Benzhydryl, Allyl, Propargyl, Methoxymethyl, $C_1$-$C_6$-Alkanoyloxy-$C_1$-$C_6$-alkyl, $C_1$-$C_6$-Alkoxycarbonyloxy-$C_1$-$C_6$-alkyl, Phthalidyl oder 5-Methy1-1,3-dioxolen-2-on-4-yl-methyl bedeutet und in der die $R^2$-O-Gruppe in syn-Position steht.

2. Verfahren zur Herstellung von Cephalosporinderivaten der allgemeinen Formel I und ihrer physiologisch verträglichen Säureadditionssalze, dadurch gekennzeichnet, daß man

a) eine Verbindung der allgemeinen Form II

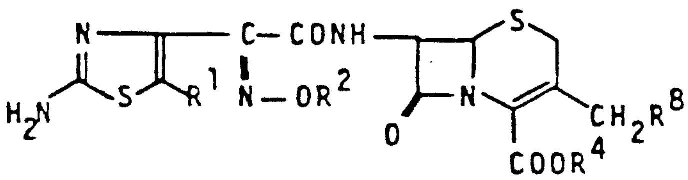

(II)

oder deren Salze, worin $R^1$, $R^2$ und $R^4$ die in Formel I genannte Bedeutung haben, die Aminogruppe auch geschützt sein kann, und $R^8$ eine durch Imidazol oder dasjenige Imidazolderivat, das den Resten $R^3$ der Formel I entspricht, austauschbare Gruppe bedeutet, mit Imidazol oder diesem Imidazolderivat umsetzt,

α) eine gegebenenfalls vorhandene Schutzgruppe abspaltet und

β) falls erforderlich, das erhaltene Produkt in ein physiologisch verträgliches Säureadditionssalz überführt.

oder

b) eine Verbindung der allgemeinen Formel III

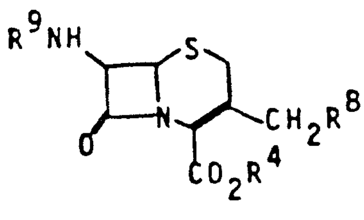

(III)

worin $R^4$ und $R^8$ die vorstehend für die Formel II genannte Bedeutung hat und $R^9$ für Wasserstoff oder eine Aminoschutzgruppe steht, mit Imidazol oder Imidazolderivat, das dem in Formel I definierten Rest $R^3$ zugrunde liegt, umsetzt unter Bildung der Verbindung der allgemeinen Formel

IV,

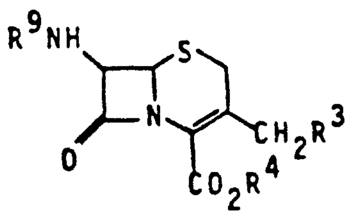

(IV)

in der $R^3$, $R^4$ und $R^9$ die oben genannte Bedeutung haben und

α) eine gegebenenfalls vorhandene Aminoschutzgruppe abspaltet und

β) die Verbindung IV, worin $R^9$ Wasserstoff bedeutet, entweder als solche oder in Form eines reaktionsfähigen Derivates mit einer 2-syn-Oxyiminoessigsäure der allgemeinen Formel V,

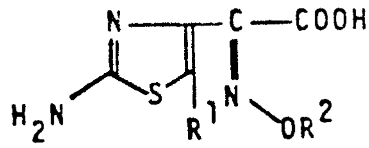

(V)

worin $R^1$ und $R^2$ die genannte Bedeutung besitzen und die Aminogruppe auch in geschützter Form vorliegen kann, oder mit einem an der Carboxylgruppe aktivierten Derivat dieser Verbindung umsetzt und

α) eine gegebenenfalls vorhandene Schutzgruppe abspaltet und

β) falls erforderlich, das erhaltene Produkt der allgemeinen Formel I in ein physiologisch verträgliches Säureadditionssalz überfuhrt und γ) - falls Ester erhalten werden sollen - die nach den Verfahrensvarianten a) oder b) erhaltenen Verbindungen der Formel I in der $R^4$ für Wasserstoff steht, in in sich bekannter Weise in die unter $R^4$ angegebenen Ester überführt.

**3.** Gegen bakterielle Infektionen wirksame pharmazeutische Präparate, gekennzeichnet durch einen Gehalt in Cephalosporinderivaten der allgemeinen Formel I.

**4.** Verfahren zur Herstellung von gegen bakterielle Infektionen wirksamen pharmazeutischen Präparaten, dadurch gekennzeichnet, daß ein Cephalosporinderivat der allgemeinen Formel I gegebenenfalls mit pharmazeutisch üblichen Trägerstoffen oder Verdünnungsmitteln in eine pharmazeutisch geeignete Verabreichungsform gebracht wird.

**5.** Verwendung von Cephalosporinderivaten der allgemeinen Formel I zur Herstellung von pharmazeutischen Präparaten zur Bekämpfung bakterieller Infektionen.

**Patentansprüche für folgenden Vertragsstaat : AT**

**1.** Verfahren zur Herstellung von Cephalosporinderivaten der allgemeinen Formel I

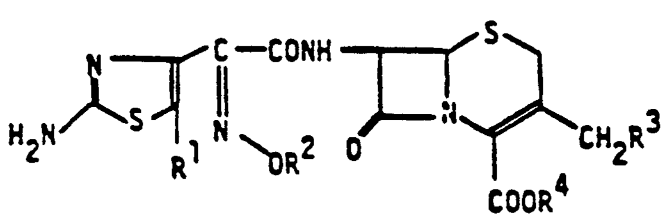

(I)

und deren physiologisch verträglichen Säureadditionssalzen, worin

$R^1$ Wasserstoff oder Fluor, Chlor oder Brom

$R^2$ Wasserstoff,

$C_1$-$C_6$-Alkyl,das ein- oder mehrfach, gleich oder verschieden substituiert sein kann durch Fluor, Phenyl, 2- der 3- oder 4-Tolyl, 2- oder 3- oder 4-Chlorphenyl, 1,3-Thiazol-4-yl, Imidazol-1-yl, $C_1$-$C_4$-Alkylthio, $C_1$-$C_4$-Alkyloxy, Nitril oder Carbamoyl, worin die Aminogruppe ein- oder zweifach, gleich oder verschieden substituiert sein kann durch $C_1$-$C_6$-Alkyl, Hydroxy-($C_1$-$C_2$)-alkyl, Hydroxy oder Methoxy;

$C_2$-$C_6$-Alkenyl, das gegebenenfalls ein- oder mehrfach, gleich oder verschieden substituiert sein kann durch Chlor oder Brom,

$C_2$-$C_6$-Alkinyl, das gegebenenfalls substituiert sein kann durch Halogen,

$C_3$-$C_7$-Cycloalkyl, das gegebenenfalls substituiert sein kann durch Halogen,

$C_4$-$C_7$-Cycloalkenyl,

$C_3$-$C_7$-Cycloalkylmethyl,

die Gruppen $-CH_2=CH-R^7$ oder

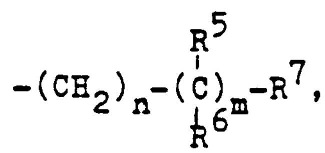

worin

m und n jeweils für 0 oder 1 steht,

$R^5$ und $R^6$ gleich oder verschieden sein können und Wasserstoff, Phenyl oder eine $C_1$-$C_{11}$-Alkylgruppe bedeuten, oder zusammen mit dem Kohlenstoff, an das sie gebunden sind, eine Methylen- oder eine $C_3$-$C_7$-Cycloalkylidengruppe bilden,

$R^7$ eine HOOC- oder $C_1$-$C_4$-Alkyl-OOC-Gruppe

$R^3$ einen Imidazol-1-yl-Rest

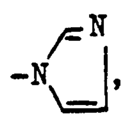

der ein- oder mehrfach, gleich oder verschieden substituiert sein kann durch $C_1$-$C_6$-Alkyl, das ein- oder mehrfach substituiert sein kann durch Hydroxy, Acetoxy, Carbamoyloxy, Chlor, Carboxy, $C_1$-$C_6$-Alkyloxycarbonyl, Formyl, $C_1$-$C_6$-Alkylcarbonyl, Cyano, Carbamoyl, $C_1$-$C_6$-Alkoxy und wobei 2 benachbarte Alkylgruppen auch zu einem gegebenenfalls durch $C_1$-$C_6$-Alkyl, Halogen, Hydroxy, Oxo, Carboxy, Carbamoyl substituierten Di- bis Decamethylen-Ring geschlossen sein können, in dem ein C-Atom durch eine Sauerstoff- oder Schwefelatom ersetzt sein kann und auch eine oder zwei Doppelbindungen enthalten sein können,

durch $C_2$-$C_6$-Alkenyl, das durch Hydroxy substituiert sein kann,

durch $C_2$-$C_6$-Alkinyl,

durch $C_3$-$C_6$-Cycloalkyl

durch $C_3$-$C_6$-Cycloalkylmethyl

durch Phenyl oder Benzyl, die durch $C_1$-$C_4$-Alkyl, Fluor, Chlor, Hydroxy, $C_1$-$C_4$-Alkyloxy substituiert sein können,

durch $C_1$-$C_6$-Alkoxy oder durch $C_1$-$C_6$-Alkylthio,

durch Fluor, Chlor, Brom, Jod, Trifluormethyl, Cyano, Hydroxy oder Mercapto,

durch Carboxy, $C_1$-$C_6$-Alkoxycarbonyl oder Carbamoyl, das am Stickstoff ein- oder zweimal substituiert sein kann durch $C_1$-$C_6$-Alkyl, Hydroxy oder Methoxy,

durch Carbazoyl, das am Stickstoff ein- oder zweimal substituiert sein kann durch $C_1$-$C_4$-Alkyl,

durch Nitro, Amino oder Di-($C_1$-$C_2$)-alkylamino

durch Formyl, $C_1$-$C_4$-Alkylcarbonyl oder Benzoyl,

$R^4$ Wasserstoff, $C_1$-$C_6$-Alkyl, Benzhydryl, Allyl, Propargyl, Methoxymethyl, $C_1$-$C_6$-Alkanoyloxy-

$C_1$-$C_6$-alkyl, $C_1$-$C_6$-Alkoxycarbonyloxy-$C_1$-$C_6$-alkyl, Phthalidyl oder 5-Methyl-1,3-dioxolen-2-on-4-yl-methyl bedeutet und in der die $R^2$-O-Gruppe in syn-Position steht,

dadurch gekennzeichnet, daß man

a) eine Verbindung der allgemeinen Form II

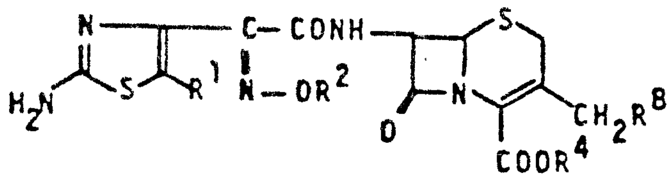

(II)

oder deren Salze, worin $R^1$, $R^2$ und $R^4$ die in Formel I genannte Bedeutung haben, die Aminogruppe auch geschützt sein kann, und $R^8$ eine durch Imidazol oder dasjenige Imidazolderivat, das den Resten $R^3$ der Formel I entspricht, austauschbare Gruppe bedeutet, mit Imidazol oder diesem Imidazolderivat umsetzt,

α) eine gegebenenfalls vorhandene Schutzgruppe abspaltet und

β) falls erforderlich, das erhaltene Produkt in ein physiologisch verträgliches Säureadditionssalz überführt.

oder

b) eine Verbindung der allgemeinen Formel III

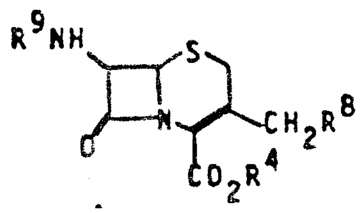

(III)

worin $R^4$ und $R^8$ die vorstehend für die Formel II genannte Bedeutung hat und $R^9$ für Wasserstoff oder eine Aminoschutzgruppe steht, mit Imidazol oder dem Imidazolderivat, das dem in Formel I definierten Rest $R^3$ zugrunde liegt, umsetzt unter Bildung der Verbindung der allgemeinen Formel IV,

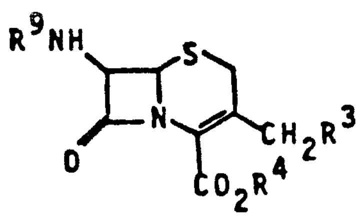

(IV)

in der $R^3$, $R^4$ und $R^9$ die oben genannte Bedeutung haben und

α) eine gegebenenfalls vorhandene Aminoschutzgruppe abspaltet und

β) die Verbindung IV, worin $R^9$ Wasserstoff bedeutet, entweder als solche oder in Form eines reaktionsfähigen Derivates mit einer 2-syn-Oxyiminoessigsäure der allgemeinen Formel V,

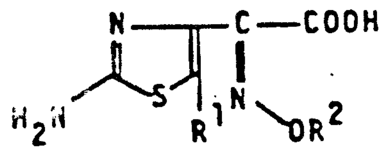

(V)

worin $R^1$ und $R^2$ die genannte Bedeutung besitzen und die Aminogruppe auch in geschützter Form vorliegen kann, oder mit einem an der Carboxylgruppe aktivierten Derivat dieser Verbindung umsetzt und

α) eine gegebenenfalls vorhandene Schutzgruppe abspaltet und

β) falls erforderlich, das erhaltene Produkt der allgemeinen Formel I in ein physiologisch verträgliches Säureadditionssalz überführt und

γ) - falls Ester erhalten werden sollen - die nach den Verfahrensvarianten a) oder b) erhaltenen Verbindungen der Formel I in der $R^4$ für Wasserstoff steht, in an sich bekannter Weise in die unter $R^4$ angegebenen Ester überführt.

**2.** Verfahren zur Herstellung von gegen bakterielle Infektionen wirksamen pharmazeutischen Präparaten, dadurch gekennzeichnet, daß ein Cephalosporinderivat der allgemeinen Formel I gegebenenfalls mit pharmazeutisch üblichen Trägerstoffen oder Verdünnungsmitteln in eine pharmazeutisch geeignete Verabreichungsform gebracht wird.

## Claims

### Claims for following the Contracting States : BE, CH, DE, FR, GB, IT, LI, LU, NL, SE

**1.** A cephalosporin derivative of the formula I

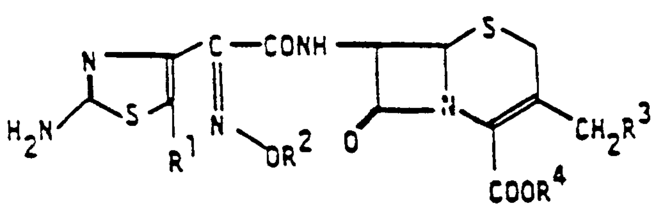

(I)

or a physiologically acceptable acid addition salt thereof, in which

$R^1$ denotes hydrogen or fluorine, chlorine or bromine,

$R^2$ denotes hydrogen,

$C_1$-$C_6$-alkyl, which can be mono- or polysubstituted by identical or different substituents from the group comprising fluorine, phenyl, 2- or 3- or 4-tolyl, 2- or 3- or 4-chlorophenyl, 1,3-thiazol-4-yl, imidazol-1-yl, $C_1$-$C_4$-alkylthio, $C_1$-$C_4$-alkoxy, nitrile and carbamyl, in which the amino group can be mono- or disubstituted by identical or different substituents from the group comprising $C_1$-$C_6$-alkyl, hydroxy-($C_1$-$C_2$)-alkyl, hydroxyl and methoxy, $C_2$-$C_6$-alkenyl, which can optionally be mono- or poly-substituted by identical or different substituents from the group comprising chlorine and bromine,

$C_2$-$C_6$-alkynyl, which can be optionally substituted by halogen,

$C_3$-$C_7$-cycloalkyl, which can be optionally substituted by halogen,

$C_4$-$C_7$-cycloalkenyl

$C_3$-$C_7$-cycloalkylmethyl,

the group $-CH_2=CH-R^7$ or

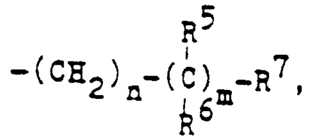

in which m and n each represent 0 or 1 and $R^5$ and $R^6$ can be identical or different and denote hydrogen, phenyl or a $C_1$-$C_{11}$-alkyl group, or together with the carbon to which they are bonded form a methylene or a $C_3$-$C_7$-cycloalkylidene group,
$R^7$ denotes an HOOC- or $C_1$-$C_4$-alkyl-OOC- group,
$R^3$ denotes an imidazol-1-yl radical

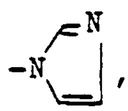

which can be mono- or polysubstituted by identical or different substituents from the group comprising $C_1$-$C_6$-alkyl, which can be mono-or polysubstituted by hydroxyl, acetoxy, carbamyloxy, chlorine, carboxyl, $C_1$-$C_6$-alkoxycarbonyl, formyl, $C_1$-$C_6$-alkylcarbonyl, cyano, carbamyl and $C_1$-$C_6$-alkoxy and in which 2 adjacent alkyl groups can also be closed to form a di- to decamethylene ring which is optionally substituted by $C_1$-$C_6$-alkyl, halogen, hydroxyl, oxo, carboxyl or carbamyl and in which one carbon atom can be replaced by an oxygen or sulfur atom and which can also contain one or two double bonds,
or by $C_2$-$C_6$-alkenyl, which can be substituted by hydroxyl,
by $C_2$-$C_6$-alkynyl,
by $C_3$-$C_6$-cycloalkyl,
by $C_3$-$C_6$-cycloalkylmethyl,
by phenyl or benzyl, which can be substituted by $C_1$-$C_4$-alkyl, fluorine, chlorine, hydroxyl or $C_1$-$C_4$-alkoxy,
by $C_1$-$C_6$-alkoxy or by $C_1$-$C_6$-alkylthio,
by fluorine, chlorine, bromine, iodine, trifluoromethyl, cyano, hydroxyl or mercapto,
by carboxyl, $C_1$-$C_6$-alkoxycarbonyl or carbamyl, which can be mono- or disubstituted on the nitrogen by $C_1$-$C_6$-alkyl, hydroxyl or methoxy; by carbazoyl, which can be mono- or disubstituted on the nitrogen by $C_1$-$C_4$-alkyl,
by nitro, amino or di-($C_1$-$C_2$)-alkylamino,
or by formyl, $C_1$-$C_4$-alkylcarbonyl or benzoyl,
$R^4$ denotes hydrogen, $C_1$-$C_6$-alkyl, benzhydryl, allyl, propargyl, methoxymethyl, $C_1$-$C_6$-alkanoyloxy-$C_1$-$C_6$-alkyl, $C_1$-$C_6$-alkoxycarbonyloxy-$C_1$-$C_6$-alkyl, phthalidyl or 5-methyl-1,3-dioxolen-2-on-4-yl-methyl, and in which the $R^2$-O- group is in the syn-position.

**2.** A process for the preparation of a cephalosporin derivative of the formula I or a physiologically acceptable acid addition salt thereof, which comprises

a) reacting a compound of the formula II

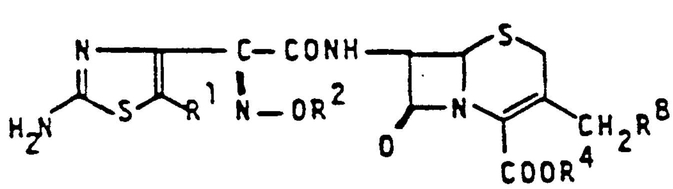

(II)

or a salt thereof, in which $R^1$, $R^2$ and $R^4$ have the meaning given in formula I, the amino group can also be protected and $R^8$ denotes a group which can be replaced by imidazole or that imidazole derivative which corresponds to the radicals $R^3$ of formula I, with imidazole or this imidazole derivative,

α) splitting off any protective group which may be present and

β) if necessary, converting the resulting product into a physiologically acceptable acid addition salt,

or

b) reacting a compound of the formula III

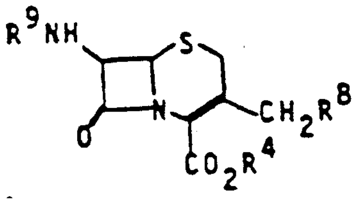

(III)

in which $R^4$ and $R^8$ have the meaning given above for formula II and $R^9$ represents hydrogen or an amino-protective group, with imidazole or the imidazole derivative on which the radical $R^3$ defined in formula I is based, to form the compound of the formula IV

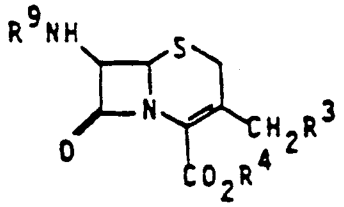

(IV)

in which $R^3$, $R^4$ and $R^9$ have the abovementioned meaning and

α) splitting off any amino-protective group which may be present and

β) reacting the compound IV in which $R^9$ denotes hydrogen, either as such or in the form of a reactive derivative, with a 2-syn-oxyiminoacetic acid of the general formula V

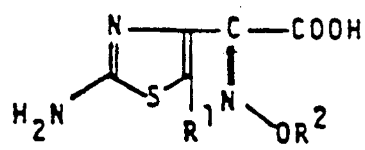

(V)

in which $R^1$ and $R^2$ have the meaning given and the amino group can also be in the protected form, or with a derivative of this compound activated on the carboxyl group and

α) splitting off any protective group which may be present,

β) if necessary, converting the resulting product of the formula I into a physiologically acceptable acid addition salt and

γ) - if an ester is to be obtained - the compound of the formula I in which $R^4$ represents hydrogen obtained by process variant a) or b) is converted into the ester mentioned under $R^4$ in a manner which is known per se.

**3.** A pharmaceutical product which is active against bacterial infections, which contains a cephalosporin derivative of the formula I.

**4.** A process for the preparation of a pharmaceutical product which is active against bacterial infections, which comprises bringing a cephalosporin derivative of the formula I into a pharmaceutically suitable administration form, if appropriate with pharmaceutically customary excipients or diluents.

**5.** The use of a cephalosporin derivative of the formula I for preparing pharmaceutical products to control bacterial infections.

**Claims for the following Contracting State : AT**

**1.** A process for the preparation of a cephalosporin derivative of the formula I

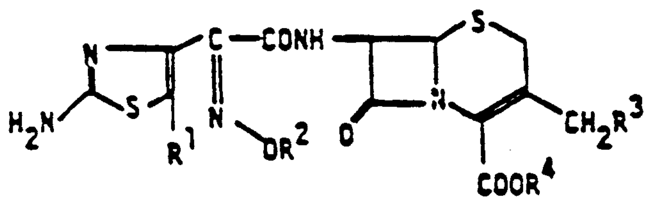

(I)

or a physiologically acceptable acid addition salt thereof, in which
$R^1$ denotes hydrogen or fluorine, chlorine or bromine,
$R^2$ denotes hydrogen,
$C_1$-$C_6$-alkyl, which can be mono-or polysubstituted by identical or different substituents from the group comprising fluorine, phenyl, 2- or 3- or 4-tolyl, 2- or 3- or 4-chlorophenyl, 1,3-thiazol-4-yl, imidazol-1-yl, $C_1$-$C_4$-alkylthio, $C_1$-$C_4$-alkoxy, nitrile and carbamyl, in which the amino group can be mono- or disubstituted by identical or different substituents from the group comprising $C_1$-$C_6$-alkyl, hydroxy-($C_1$-$C_2$)-alkyl, hydroxyl and methoxy, $C_2$-$C_6$-alkenyl, which can optionally be mono-or polysubstituted by identical or different substituents from the group comprising chlorine and bromine,
$C_2$-$C_6$-alkynyl, which can be optionally substituted by halogen,
$C_3$-$C_7$-cycloalkyl, which can be optionally substituted by halogen,
$C_4$-$C_7$-cycloalkenyl,
$C_3$-$C_7$-cycloalkylmethyl,
the group $-CH_2=CH-R^7$ or

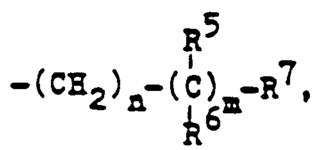

in which m and n each represent 0 or 1 and
$R^5$ and $R^6$ can be identical or different and denote hydrogen, phenyl or a $C_1$-$C_{11}$-alkyl group, or together with the carbon to which they are bonded form a methylene or a $C_3$-$C_7$-cycloalkylidene group,
$R^7$ denotes an HOOC- or $C_1$-$C_4$-alkyl-OOC- group,
$R^3$ denotes an imidazol-1-yl radical

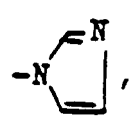

which can be mono- or polysubstituted by identical or different substituents from the group comprising $C_1$-$C_6$-alkyl, which can be mono- or polysubstituted by hydroxyl, acetoxy, carbamyloxy, chlorine, carboxyl, $C_1$-$C_6$-alkoxycarbonyl, formyl, $C_1$-$C_6$-alkylcarbonyl, cyano, carbamyl and $C_1$-$C_6$-alkoxy and in which 2 adjacent alkyl groups can also be closed to form a di- to decamethylene ring which is optionally substituted by $C_1$-$C_6$-alkyl, halogen, hydroxyl, oxo, carboxyl or carbamyl and in which one carbon atom can be replaced by an oxygen or sulfur atom and which can also contain one or two double bonds,
or by $C_2$-$C_6$-alkenyl, which can be substituted by hydroxyl,
by $C_2$-$C_6$-alkynyl,
by $C_3$-$C_6$-cycloalkyl,
by $C_3$-$C_6$-cycloalkylmethyl,
by phenyl or benzyl, which can be substituted by $C_1$-$C_4$-alkyl, fluorine, chlorine, hydroxyl or $C_1$-$C_4$-alkyloxy,
by $C_1$-$C_6$-alkoxy or by $C_1$-$C_6$-alkylthio,

by fluorine, chlorine, bromine, iodine, trifluoromethyl, cyano, hydroxyl or mercapto,
by carboxyl, $C_1$-$C_6$-alkoxycarbonyl or carbamyl, which can be mono- or disubstituted on the nitrogen by $C_1$-$C_6$-alkyl, hydroxyl or methoxy; by carbazoyl, which can be mono- or disubstituted on the nitrogen by $C_1$-$C_4$-alkyl,
by nitro, amino or di-($C_1$-$C_2$)-alkylamino,
or by formyl, $C_1$-$C_4$-alkylcarbonyl or benzoyl,
$R^4$ denotes hydrogen, $C_1$-$C_6$-alkyl, benzhydryl, allyl, propargyl, methoxymethyl, $C_1$-$C_6$-alkanoyloxy-$C_1$-$C_6$-alkyl, $C_1$-$C_6$-alkoxycarbonyloxy-$C_1$-$C_6$-alkyl, phthalidyl or 5-methyl-1,3-dioxolen-2-on-4-yl-methyl, and in which the $R^2$-O-group is in the syn-position, which comprises

a) reacting a compound of the formula II

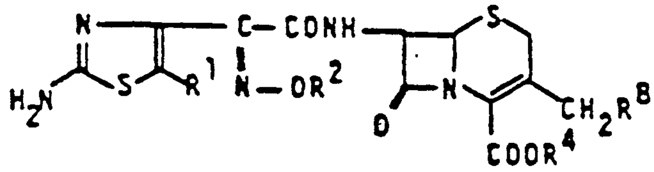

(II)

or a salt thereof, in which $R^1$, $R^2$ and $R^4$ have the meaning given in formula I, the amino group can also be protected and $R^8$ denotes a group which can be replaced by imidazole or that imidazole derivative which corresponds to the radicals $R^3$ of formula I, with imidazole or this imidazole derivative,

α) splitting off any protective group which may be present and

β) if necessary, converting the resulting product into a physiologically acceptable acid addition salt,

or

b) reacting a compound of the formula III

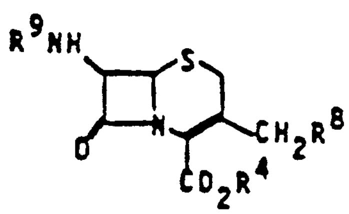

(III)

in which $R^4$ and $R^8$ have the meaning given above for formula II and $R^9$ represents hydrogen or an amino-protective group, with imidazole or the imidazole derivative on which the radical $R^3$ defined in formula I is based, to form the compound of the formula IV

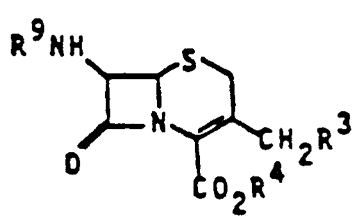

(IV)

in which $R^3$, $R^4$ and $R^9$ have the abovementioned meaning and

α) splitting off any amino-protective group which may be present and

$\beta$) reacting the compound IV in which $R^9$ denotes hydrogen, either as such or in the form of a reactive derivative, with a 2-syn-oxyiminoacetic acid of the general formula V

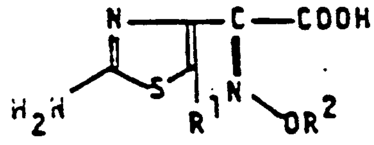

(V)

in which $R^1$ and $R^2$ have the meaning given and the amino group can also be in the protected form, or with a derivative of this compound activated on the carboxyl group and
$\alpha$) splitting off any protective group which may be present,
$\beta$) if necessary, converting the resulting product of the formula I into a physiologically acceptable acid addition salt and
$\gamma$) - if an ester is to be obtained - the compound of the formula I in which $R^4$ represents hydrogen obtained by process variant a) or b) is converted into the ester mentioned under $R^4$ in a manner which is known per se.

**2.** A process for the preparation of a pharmaceutical product which is active against bacterial infections, which comprises bringing a cephalosporin derivative of the formula I into a pharmaceutically suitable administration form, if appropriate with pharmaceutically customary excipients or diluents.

**Revendications**

**Revendications pour l'Etat contractant suivant : BE, CH, DE, FR, GB IT, LI, LU, NL, SE**

**1.** Dérivés de céphalosporine de formule générale I

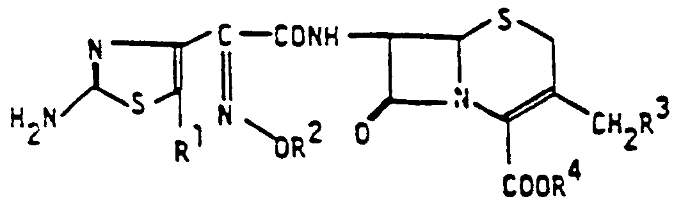

(I)

et sels d'addition avec des acides physiologiquement acceptables de ceux-ci, formule dans laquelle

$R^1$ représente un atome d'hydrogène ou de fluor, chlore, ou brome;

$R^2$ représente un atome d'hydrogène,
un radical alkyle en $C_1$-$C_6$ qui peut être une ou plusieurs fois substitué, de façon identique ou différente, par un ou des atomes de fluor ou groupes phényle, 2- ou 3- ou 4-tolyle, 2- ou 3- ou 4-chlorophényle, 1,3-thiazol-4-yle, imidazol-1-yle, alkyl($C_1$-$C_4$)-thio, alcoxy en $C_1$-$C_4$, nitrile ou carbamoyle, dans lequel le groupe amino peut être une ou deux fois substitué, de façon identique ou différente, par un ou des groupes alkyle en $C_1$-$C_6$, hydroxyalkyle en $C_1$-$C_2$, hydroxy ou méthoxy;
un radical alcényle en $C_2$-$C_6$ qui peut éventuellement être une ou plusieurs fois substitué, de façon identique ou différente, par un ou des atomes de chlore ou de brome;
un radical alcynyle en $C_2$-$C_6$, qui peut éventuellement être substitué par un atome d'halogène;
un radical cycloalkyle en $C_3$-$C_7$, qui peut éventuellement être substitué par un atome d'halogène;
un radical cycloalcényle en $C_4$-$C_7$;
un radical cycloalkyl($C_3$-$C_7$)-méthyle;
le groupe $-CH_2=CH-R^7$ ou

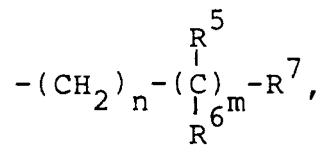

dans lequel m et n sont chacun 0 ou 1, et

$R^5$ et $R^6$ peuvent être identiques ou différents et représentent un atome d'hydrogène, le groupe phényle ou un groupe alkyle en $C_1$-$C_{11}$, ou forment ensemble, conjointement avec l'atome de carbone auquel ils sont liés, un groupe méthylène ou un groupe cycloalkylidène en $C_3$-$C_7$,$R^7$ représente un groupe HOOC- ou alkyl($C_1$-$C_4$)-OOC-;

$R^3$ représente un radical imidazol-1-yle

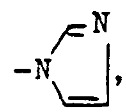

qui peut être une ou plusieurs fois substitué, de façon identique ou différente, par un groupe alkyle en $C_1$-$C_6$ qui peut être une ou plusieurs fois substitué par un ou des atomes de chlore ou groupes hydroxy, acétoxy, carbamoyloxy, carboxy, alkyl($C_1$-$C_6$)-oxycarbonyle, formyle, alkyl($C_1$-$C_6$)-carbonyle, cyano, carbamoyle, alkyloxy en $C_1$-$C_6$, et deux groupes alkyle adjacents pouvant également être cyclisés en un cycle di- à décaméthylène éventuellement substitué par un atome d'halogène ou un groupe alkyle en $C_1$-$C_6$, hydroxy, oxo, carboxy, carbamoyle, cycle dans lequel un atome de carbone peut être remplacé par un atome d'oxygène ou de soufre, et une ou deux doubles liaisons peuvent également être contenues,

par un groupe alcényle en $C_2$-$C_6$ qui peut être substitué par le groupe hydroxy,

par un groupe alcynyle en $C_2$-$C_6$,

par un groupe cycloalkyle en $C_3$-$C_6$,

par un groupe cycloalkyl($C_3$-$C_6$)-méthyle,

par un groupe phényle ou benzyle qui peut être substitué par un atome de chlore ou de fluor ou un groupe alkyle en $C_1$-$C_4$, hydroxy, alkyloxy en $C_1$-$C_4$,

par un groupe alcoxy en $C_1$-$C_6$ ou par un groupe alkyl($C_1$-$C_6$)-thio,

par un atome de fluor, chlore, brome, iode, le groupe trifluorométhyle, cyano, hydroxy ou mercapto,

par un groupe carboxy, alcoxy($C_1$-$C_6$)-carbonyle ou carbamoyle qui peut être une ou deux fois substitué à l'azote par un ou des groupes alkyle en $C_1$-$C_6$, hydroxy ou méthoxy,

par un groupe carbazoyle qui peut être une ou deux fois substitué à l'azote par un groupe alkyle en $C_1$-$C_4$,

par un groupe nitro, amino ou dialkyl($C_1$-$C_2$)-amino,

par un groupe formyle, alkyl($C_1$-$C_4$)-carbonyle ou benzoyle;

$R^4$ représente un atome d'hydrogène ou un radical alkyle en $C_1$-$C_6$, benzhydryle, allyle, propargyle, méthoxyméthyle, alcanoyloxy($C_1$-$C_6$)-alkyle($C_1$-$C_6$), alcoxy($C_1$-$C_6$)-carbonyloxy-alkyle($C_1$-$C_6$), phtalidyle ou 5-méthyl-1,3-dioxolène-2-one-4-yl-méthyle,

et dans laquelle le groupe $R^2O$ est en position syn.

**2.** Procédé pour la préparation de dérivés de céphalosporine de formule générale I et de leurs sels d'addition avec des acides physiologiquement acceptables, caractérisé en ce que

a) on fait réagir un composé de formule générale II

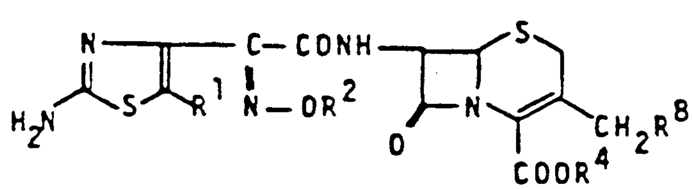

(II)

ou l'un de ses sels, dans lequel $R^1$, $R^2$ et $R^4$ ont les significations données dans la formule I, le

groupe amino peut également être protégé, et $R^8$ représente un groupe remplaçable par l'imidazole ou le dérivé d'imidazole qui correspond aux radicaux $R^3$ de la formule I,
avec l'imidazole ou ce dérivé d'imidazole,

α) on élimine un groupe protecteur éventuellement présent, et

β) si nécessaire, on convertit le produit obtenu en un sel d'addition avec un acide physiologiquement acceptable,

ou

b) on fait réagir un composé de formule générale III

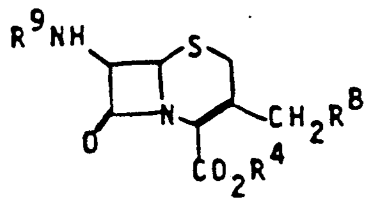

(III)

dans laquelle $R^4$ et $R^8$ ont les significations données précédemment à propos de la formule II, et $R^9$ représente un atome d'hydrogène ou un groupe protecteur de fonction amino,
avec l'imidazole ou le dérivé d'imidazole qui est à la base du radical $R^3$ défini dans la formule I, avec formation du composé de formule générale IV

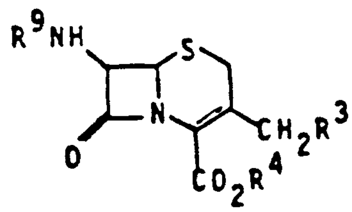

(IV)

dans laquelle $R^3$, $R^4$ et $R^9$ ont les significations données plus haut, et

α) on élimine un groupe protecteur de fonction amino éventuellement présent, et

β) on fait réagir le composé IV, dans lequel $R^9$ représenteun atome d'hydrogène, soit tel quel, soit sous forme d'un dérivé réactif, avec un acide 2-syn-oxyiminoacétique de formule générale V

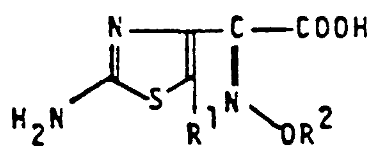

(V)

dans laquelle $R^1$ et $R^2$ ont les significations données et le groupe amino peut également se trouver sous forme protégée, ou avec un dérivé de ce composé, activé au niveau du groupe carboxy, et

α) on élimine un groupe protecteur éventuellement présent,

β) si nécessaire, on convertit le produit obtenu de formule générale I en un sel d'addition avec un acide physiologiquement acceptable, et

γ) - dans le cas où l'on désire obtenir des esters - on convertit en les esters indiqués en $R^4$, d'une façon connue en soi, les composés de formule I, obtenus selon la variante a) ou b) du procédé, dans lesquels $R^4$ représente un atome d'hydrogène.

**3.** Compositions pharmaceutiques efficaces contre des infections bactériennes, caractérisées par une teneur en dérivés de céphalosporine de formule générale I.

**4.** Procédé pour la préparation de compositions pharmaceutiques efficaces contre des infections bactériennes, caractérisé en ce que l'on met sous une forme pharmaceutique appropriée un dérivé de céphalosporine de formule générale I, éventuellement avec des véhicules ou diluants pharmaceutiquement usuels.

**5.** Utilisation de dérivés de céphalosporine de formule générale I pour la préparation de compositions pharmaceutiques pour la lutte contre des infections bactériennes.

**Revendications pour l'Etat contractant suivant : AT**

**1.** Procédé pour la préparation de dérivés de céphalosporine de formule générale I

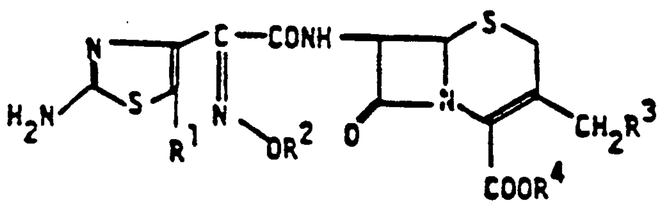

(I)

et de leurs sels d'addition avec des acides physiologiquement acceptables, formule dans laquelle

$R^1$ représente un atome d'hydrogène ou de fluor, chlore, ou brome;

$R^2$ représente un atome d'hydrogène,
un radical alkyle en $C_1$-$C_6$ qui peut être une ou plusieurs fois substitué, de façon identique ou différente, par un ou des atomes de fluor ou groupes phényle, 2- ou 3- ou 4-tolyle, 2- ou 3- ou 4-chlorophényle, 1,3-thiazol-4-yle, imidazol-1-yle, alkyl($C_1$-$C_4$)-thio, alcoxy en $C_1$-$C_4$, nitrile ou carbamoyle, dans lequel le groupe amino peut être une ou deux fois substitué, de façon identique ou différente, par un ou des groupes alkyle en $C_1$-$C_6$, hydroxyalkyle en $C_1$-$C_2$, hydroxy ou méthoxy;
un radical alcényle en $C_2$-$C_6$ qui peut éventuellement être une ou plusieurs fois substitué, de façon identique ou différente, par un ou des atomes de chlore ou de brome;
un radical alcynyle en $C_2$-$C_6$, qui peut éventuellement être substitué par un atome d'halogène;
un radical cycloalkyle en $C_3$-$C_7$, qui peut éventuellement être substitué par un atome d'halogène;
un radical cycloalcényle en $C_4$-$C_7$;
un radical cycloalkyl($C_3$-$C_7$)-méthyle;
le groupe $-CH_2=CH-R^7$ ou

$$-(CH_2)_n-(\overset{R^5}{\underset{R^6}{C}})_m-R^7,$$

dans lequel m et n sont chacun 0 ou 1, et
$R^5$ et $R^6$ peuvent être identiques ou différents et représentent un atome d'hydrogène, le groupe phényle ou un groupe alkyle en $C_1$-$C_{11}$, ou forment ensemble, conjointement avec l'atome de carbone auquel ils sont liés, un groupe méthylène ou un groupe cycloalkylidène en $C_3$-$C_7$,
$R^7$ représente un groupe HOOC- ou alkyl($C_1$-$C_4$)-OOC-;

$R^3$ représente un radical imidazol-1-yle

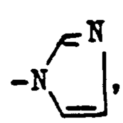

qui peut être une ou plusieurs fois substitué, de façon identique ou différente, par un groupe alkyle en $C_1$-$C_6$ qui peut être une ou plusieurs fois substitué par un ou des atomes de chlore ou groupes hydroxy, acétoxy, carbamoyloxy, carboxy, alkyl($C_1$-$C_6$)-oxycarbonyle, formyle, alkyl($C_1$-$C_6$)-carbonyle, cyano, carbamoyle, alkyloxy en $C_1$-$C_6$, et deux groupes alkyle adjacents pouvant également être cyclisés en un cycle di- à décaméthylène éventuellement substitué par un atome d'halogène ou un groupe alkyle en $C_1$-$C_6$, hydroxy, oxo, carboxy, carbamoyle, cycle dans lequel un atome de carbone peut être remplacé par un atome d'oxygène ou de soufre, et une ou deux doubles liaisons peuvent également être contenues,
par un groupe alcényle en $C_2$-$C_6$ qui peut être substitué par le groupe hydroxy,
par un groupe alcynyle en $C_2$-$C_6$,
par un groupe cycloalkyle en $C_3$-$C_6$,
par un groupe cycloalkyl($C_3$-$C_6$)-méthyle,
par un groupe phényle ou benzyle qui peut être substitué par un atome de chlore ou de fluor ou un groupe alkyle en $C_1$-$C_4$, hydroxy, alkyloxy en $C_1$-$C_4$,
par un groupe alcoxy en $C_1$-$C_6$ ou par un groupe alkyl-($C_1$-$C_6$)-thio,
par un atome de fluor, chlore, brome, iode, le groupe trifluorométhyle, cyano, hydroxy ou mercapto,
par un groupe carboxy, alcoxy($C_1$-$C_6$)-carbonyle ou carbamoyle qui peut être une ou deux fois substitué à l'azote par un ou des groupes alkyle en $C_1$-$C_6$, hydroxy ou méthoxy,
par un groupe carbazoyle qui peut être une ou deux fois substitué à l'azote par un groupe alkyle en $C_1$-$C_4$,
par un groupe nitro, amino ou dialkyl($C_1$-$C_2$)-amino,
par un groupe formyle, alkyl($C_1$-$C_4$)-carbonyle ou benzoyle;

$R^4$ représente un atome d'hydrogène ou un radical alkyle en $C_1$-$C_6$, benzhydryle, allyle, propargyle, méthoxyméthyle, alcanoyloxy($C_1$-$C_6$)-alkyle($C_1$-$C_6$), alcoxy($C_1$-$C_6$)-carbonyloxy-alkyle($C_1$-$C_6$), phtalidyle ou 5-méthyl-1,3-dioxolène-2-one-4-yl-méthyle,

et dans laquelle le groupe $R^2O$ est en position syn,
caractérisé en ce que

a) on fait réagir un composé de formule générale II

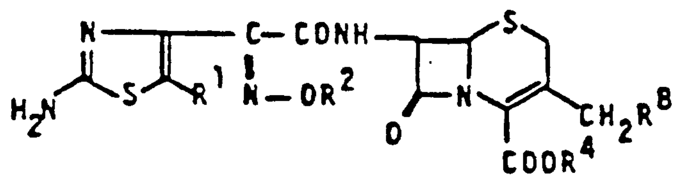

(II)

ou l'un de ses sels, dans lequel $R^1$, $R^2$ et $R^4$ ont les significations données dans la formule I, le groupe amino peut également être protégé, et $R^8$ représente un groupe remplaçable par l'imidazole ou le dérivé d'imidazole qui correspond aux radicaux $R^3$ de la formule I,
avec l'imidazole ou ce dérivé d'imidazole,

α) on élimine un groupe protecteur éventuellement présent, et

β) si nécessaire, on convertit le produit obtenu en un sel d'addition avec un acide physiologiquement acceptable,

ou

b) on fait réagir un composé de formule générale III

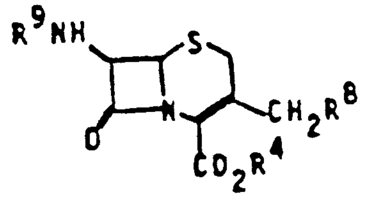

(III)

dans laquelle $R^4$ et $R^8$ ont les significations données précédemment à propos de la formule II, et $R^9$ représente un atome d'hydrogène ou un groupe protecteur de fonction amino,

avec l'imidazole ou le dérivé d'imidazole qui est à la base du radical $R^3$ défini dans la formule I, avec formation du composé de formule générale IV

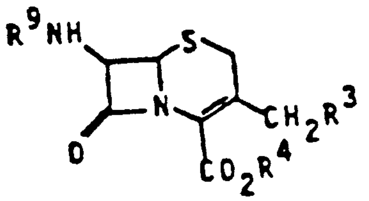

(IV)

dans laquelle $R^3$, $R^4$ et $R^9$ ont les significations données plus haut, et

α) on élimine un groupe protecteur de fonction amino éventuellement présent, et

β) on fait réagir le composé IV, dans lequel $R^9$ représenteun atome d'hydrogène, soit tel quel, soit sous forme d'un dérivé réactif, avec un acide 2-syn-oxyiminoacétique de formule générale V

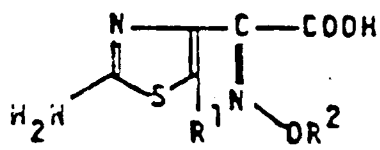

(V)

dans laquelle $R^1$ et $R^2$ ont les significations données et le groupe amino peut également se trouver sous forme protégée, ou avec un dérivé de ce composé, activé au niveau du groupe carboxy, et

α) on élimine un groupe protecteur éventuellement présent,

β) si nécessaire, on convertit le produit obtenu de formule générale I en un sel d'addition avec un acide physiologiquement acceptable, et

γ) - dans le cas où l'on désire obtenir des esters - on convertit en les esters indiqués en $R^4$, d'une façon connue en soi, les composés de formule I, obtenus selon la variante a) ou b) du procédé, dans lesquels $R^4$ représente un atome d'hydrogène.

**2.** Procédé pour la préparation de compositions pharmaceutiques efficaces contre des infections bactériennes, caractérisé en ce que l'on met sous une forme pharmaceutique appropriée un dérivé de céphalosporine de formule générale I, éventuellement avec des véhicules ou diluants pharmaceutiquement usuels.